# EUROPEAN PATENT SPECIFICATION

(11) **EP 1 296 940 B1**
(45) Date of publication and mention of the grant of the patent: **02.07.2008**
(21) Application number: 01938439.5
(22) Date of filing: 14.06.2001
(51) Int. Cl.: C07C 257/14, C07D 333/14, C07D 307/52, C07D 213/36, A61K 31/155, A61P 25/00

(54) **AMIDINE DERIVATIVES AS SELECTIVE ANTAGONISTS OF NMDA RECEPTORS**
AMIDINDERIVATE ALS SELEKTIVE NMDA-REZEPTORANTAGONISTEN
DERIVES D'AMIDINE EN TANT QU'ANTAGONISTES SELECTIFS DE RECEPTEURS NMDA

(30) Priority: 23.06.2000 GB 0015488
(43) Date of publication of application: 02.04.2003
(73) Proprietor: MERCK SHARP & DOHME LTD., Hoddesdon, Hertfordshire EN11 9BU (GB)
(72) Inventor: CURTIS, Neil Roy, Harlow, Essex CM20 2QR (GB); KULAGOWSKI, Janusz Jozef, Harlow, Essex CM20 2QR (GB); THOMAS, Steve, Harlow, Essex CM20 2QR (GB); WATT, Alan Paul, Harlow, Essex CM20 2QR (GB)
(74) Representative: Horgan, James Michael Frederic
(86) International application number: PCT/GB2001/002621
(87) International publication number: WO 2001/098262

(56) References cited:
- GB-A- 1 070 116
- GB-A- 1 131 847
- US-A- 3 476 768
- TAMIZ ET AL.: "N-(2-(4-Hydroxyphenyl)ethyl)-4-Chlorocinn amide: A novel antagonist at the 1A/2B NMDA receptor subtype" BIOORGANIC & MEDICINAL CHEMISTRY LETTERS , vol. 8, 1998, pages 199-200, XP004136845 cited in the application
- HARFENIST: "The preparation ans stereochemistry of some cinnamonitriles ..." J. AMER. CHEM. SOC., vol. 80, - 1958 pages 6261-6265, XP001018725
- MCFARLAND: "Novel anthelmintic agents." J. MED. CHEM., vol. 15, 1972, pages 365-368, XP001018691
- DATABASE CA [Online] CHEMICAL ABSTRACTS SERVICE, COLUMBUS, OHIO, US; ITO, SATORU ET AL: "Preparation of amidines as neuropeptide Y receptor antagonists and therapeutics for hyperphagia, etc." retrieved from STN Database accession no. 129:330479 CA XP002178915 & JP 10 287637 A (BANYU PHARMACEUTICAL CO., LTD., JAPAN) 27 October 1998 (1998-10-27)
- DATABASE CA [Online] CHEMICAL ABSTRACTS SERVICE, COLUMBUS, OHIO, US; MCFARLAND, JAMES W. ET AL: "Novel anthelmintic agents. IV. Noncyclic amidines related to pyrantel" retrieved from STN Database accession no. 72:55124 CA XP002178916 & J. MED. CHEM. (1970), 13(1), 109-13 ,
- DATABASE CA [Online] CHEMICAL ABSTRACTS SERVICE, COLUMBUS, OHIO, US; MCFARLAND, JAMES W.: "3-Hydroxyphenyl-substituted cyclic and acyclic amidines" retrieved from STN Database accession no. 71:91514 CA XP002178917 & ZA 6 804 589 A (PFIZER) 3 December 1968 (1968-12-03) & ZA 6 804 589 1 (PFIZER, CHAS., AND CO., INC.)
- MCFARLAND: "Novel anthelmintic agents (IV)" J. MED. CHEM., vol. 13, 1970, pages 109-113, XP001018690

## Description

The present invention relates to a class of amidine derivatives and to their use in the therapy of neurological disorders. In particular, this invention relates to amidines that are useful as selective antagonists of NR2B subunit-containing human N-methyl-D-aspartate (NMDA) receptors. The compounds of the present invention are thus useful for relieving, treating or preventing neurological and neurodegenerative disorders, including pain (and in particular neuropathic pain and headache, specifically migraine), epilepsy, stroke, anxiety, cerebral ischemia, muscular spasms, Alzheimer's Disease, Huntington's Disease and Parkinson's Disease.

Glutamate plays a key role in processes related to chronic pain and pain-associated neurotoxicity, largely acting through NMDA receptors. Much evidence points to the involvement of NMDA receptors in the development and maintenance of neuropathic pain. NMDA receptor antagonists, for example ketamine, dextromethorphan and CPP (3-(2-carboxypiperazin-4-yl)propyl-1-phosphonic acid) have been reported to produce symptomatic relief in a number of neuropathies including postherpetic neuralgia, central pain caused by spinal cord injury and phantom limb pain (Kristensen et al., Pain, 1992, 51, 249-253; Eide et al., Pain, 1995, 61, 221-228; Knox et al., Intensive Care, 1995, 23, 620-622; Max et al., Clin. Neuropharmacol., 1995, 18, 360-368). However, at analgesic doses, psychotomimetic effects that include dizziness, headache, hallucinations, dysphoria and disturbances of cognitive and motor function prohibit their widespread use. To exploit NMDA receptor antagonists as possible treatment options for neuropathic pain, it is necessary to develop new agents with a reduced side-effect profile.

Native NMDA receptors are heterodimers composed of an NMDA R1 (NR1) subunit and at least one NMDA R2 (NR2) subunit. Receptor cloning strategies have identified multiple NMDA receptor subunits in the CNS including the NR1 subfamily (with eight isoforms derived from alternative splicing of a single gene) and four NR2 subunits (A, B, C, and D) each encoded by a single gene (for review, see Whiting & Priestley, Frontiers of Neurobiology 3, Amino Acid Neurotransmission, Portland Press, 1996, 153-176). Functional receptors have different physiological and pharmacological properties and are differentially distributed in the mammalian CNS, demonstrating the functional heterogeneity of NMDA receptors (Ishii et al., J. Biol. Chem., 1993, 268, 2836-2843; Wenzel et al., NeuroReport, 1995, 7, 45-48; Laurie et al., Brain Res. Mol. Brain Res., 1997, 51, 23-32).

NR1 is found throughout the brain whereas NR2 subunits show a differential distribution. In particular, whereas NR2C is heavily expressed and NR2A is moderately expressed in the cerebellum, there is negligible expression of NR2B in this structure. Immunocytochemical studies have demonstrated a restricted distribution of the NR2B subunit, with moderate labeling of fibres in laminas I and II of the dorsal horn suggesting a presynaptic location on primary afferent fibres and possible involvement in pain transmission (Boyce et al., Neuropharmacology, 1999, 38, 611-623). The patterns of staining observed in the spinal cord, together with the data showing negligible expression of NR2B in the cerebellum, suggest that NR2B antagonists may possess antinociceptive effects, but with a reduced side effect profile in comparison to non-competitive NMDA antagonists or glycine site antagonists.

The NR1/2B selective antagonist CP-101,606 has been reported to possess antinociceptive activity in animal assays of inflammatory hyperalgesia (Taniguchi et al., Br. J. Pharmacol., 1997, 122, 809-812; Sakurada et al., Pharmacol. Biochem. Behav., 1998, 59, 339-345). In an animal assay of inflammatory hyperalgesia (carrageenan-induced mechanical hyperalgesia) the NR1/2B antagonists CP-101,606 and Ro 25-6981 have been shown to possess antinociceptive activity with a significant separation between analgesic doses and those which induced motor impairment (Boyce et al., Neuropharmacology, 1999, 38, 611-623). NR1/2B antagonists are active in a wide range of animal nociceptive assays, suggesting a clinical utility for other painful conditions in addition to those caused by nerve damage. Moreover, these compounds may have a reduced propensity to elicit the undesirable side-effects (including hallucinations, sedation and ataxia) of ketamine, dextromethorphan and other NMDA ion channel antagonists.

There is a wealth of *in vitro* and animal model data suggesting that changes in the glutamatergic system (receptors, uptake, release) increase neuronal sensitivity to previous physiological stimuli and thereby trigger secondary neuronal damage. The primary pathology underlying the generation of symptoms in Parkinson's disease is degeneration of dopaminergic neurons of the nigrostriatal pathway (Hornykiewcz, Pharmacol. Rev., 1966, 18, 925-964). Subsequent to loss of striatal dopamine, a series of changes in activity of the basal ganglia circuitry arise, including increased activity in striatal outputs to the lateral segment of the globus pallidus. Overactivity of the striatolateral pallidal pathway is thought to be responsible for the generation of parkinsonian symptoms. It has been demonstrated that selective blockade of NR2B-containing NMDA receptors with the polyamine antagonists ifenprodil and eliprodil cause a significant increase in locomotor activity in a rodent model (Nash et al., Experimental Neurology, 1999, 155, 42-48) and ifenprodil has demonstrated activity in a primate model of Parkinson's disease (Mitchell et al., Behav. Pharmacol., 1995, 6, 492-507).

The present invention provides a class of styryl amidine derivatives which are antagonists of the human NMDA receptor, being selective for those containing the NR2B subunit. As such, they will be active in the treatment of neurological and neurodegenerative disorders, especially neuropathic pain, whilst displaying fewer ataxic and related side-effects associated with other classes of NMDA receptor antagonists.

In addition, the compounds in accordance with the present invention may be useful as radioligands in assays for detecting compounds capable of binding to the NR2B subunit of the human NMDA receptor.

Bio. Med. Chem. Lett., 1998, 8, 199 discloses a series of N-(2-phenethyl)cinnamides which were assayed for antagonism at NMDA receptor subtypes, and found to be potent and selective antagonists of the NR1_{A}/2B subtype.

WO 98/37068 is stated to disclose a class of benzoic acid derivatives and related compounds for use in the treatment of arrhythmia.

The present invention provides the use of a compound of formula I, or a pharmaceutically acceptable salt or hydrate thereof: 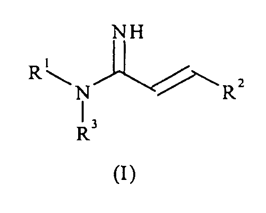 wherein:
R¹ represents C₁₋₆ alkyl, C₃₋₇cycloalkyl, C₃₋₇ cycloalkyl(C₁₋₆)alkyl, aryl, aryl(C₁₋₆)alkyl or heteroaryl(C₁₋₆)alkyl, any of which groups may be optionally substituted;
R² represents cyclopentyl, cyclohexyl, phenyl, naphthyl, pyridinyl, furyl, thienyl or pyrrolyl, any of which groups may be optionally substituted; and
R³ represents hydrogen or C₁₋₆ alkyl; or
R¹ and R³ are taken together with the intervening nitrogen atom to form an optionally substituted isoquinoline ring;
   wherein the optional substituents on R¹, R² and R³ are one or two groups independently selected from C₁₋₆alkyl, aryl, halogen, halo(C₁₋₆)alkyl, dihalo(C₁₋₆)alkyl, trihalo(C₁₋₆)alkyl, cyano, cyano(C₁₋₆)alkyl, hydroxy, hydroxymethyl, C₁₋₆alkoxy, halo(C₁₋₆)alkoxy, dihalo(C₁₋₆)alkoxy and trihalo(C₁₋₆)alkoxy;
   for the manufacture of a medicament for the treatment and/or prevention of neurological and/or neurodegenerative diseases.

The groups R¹, R² and R³ may be unsubstituted, or substituted by one or more, suitably one or two, substituents. Representative substituents include C₁₋₆ alkyl, aryl, halogen, trihalo(C₁₋₆)alkyl, hydroxy, C₁₋₆ alkoxy and trihalo(C₁₋₆)alkoxy.

As used herein, the expression "C₁₋₆ alkyl" includes methyl and ethyl groups, and straight-chained or branched propyl, butyl, pentyl and hexyl groups. Particular alkyl groups are methyl, ethyl, *n*-propyl, isopropyl, n-butyl, isobutyl, *sec*-butyl, *tert*-butyl, 3-methylbutyl and n-pentyl. Derived expressions such as "C₁₋₆ alkoxy" are to be construed accordingly. Particular values include methoxy and ethoxy.

Typical C₃₋₇ cycloalkyl groups include cyclopropyl, cyclobutyl, cyclopentyl and cyclohexyl, especially cyclohexyl.

A typical C₃₋₇ cycloalkyl(C₁₋₆)alkyl group is cyclohexylmethyl.

Typical aryl groups include phenyl and naphthyl, preferably phenyl.

The expression "aryl(C₁₋₆)alkyl" as used herein includes benzyl, phenylethyl, phenylpropyl and naphthylmethyl.

Suitable heteroaryl groups include pyridinyl, quinolinyl, isoquinolinyl, pyridazinyl, pyrimidinyl, pyrazinyl, quinoxalinyl, furyl, benzofuryl, dibenzofuryl, thienyl, benzthienyl, pyrrolyl, indolyl, pyrazolyl, indazolyl, oxazolyl, isoxazolyl, thiazolyl, isothiazolyl, imidazolyl, benzimidazolyl, oxadiazolyl, thiadiazolyl, triazolyl and tetrazolyl groups.

Suitable heteroaryl(C₁₋₆)alkyl groups include furylmethyl, furylethyl, thienylmethyl, thienylethyl, pyrazolylmethyl, pyrazolylethyl, oxazolylmethyl, isoxazolylmethyl, thiazolylmethyl, isothiazolylmethyl, imidazolylmethyl, benzimidazolylmethyl, oxadiazolylmethyl, thiadiazolylmethyl, triazolylmethyl, triazolylethyl, tetrazolylmethyl, pyridinylmethyl, pyridinylethyl, pyridazinylmethyl, pyrimidinylmethyl, pyrazinylmethyl, quinolinylmethyl and isoquinolinylmethyl.

The term "halogen" as used herein includes fluorine, chlorine, bromine and iodine, especially fluorine or chlorine.

For use in medicine, the salts of the compounds of formula I will be pharmaceutically acceptable salts. Other salts may, however, be useful in the preparation of the compounds according to the invention or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic ligands, e.g. quaternary ammonium salts.

Where the compounds according to the invention have at least one asymmetric centre, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

Suitable values for the substituent R¹ in the compounds according to the invention include methyl, ethyl, *n*-propyl, isopropyl, *n*-butyl, isobutyl, *sec*-butyl, *tert*-butyl, 3-methylbutyl, *n*-pentyl, cyclobutyl, cyclopentyl, cyclohexyl, cyclohexylmethyl, phenyl, naphthyl, benzyl, phenylethyl, phenylpropyl, pyridinyl, furyl, thienyl, pyrrolyl, indolyl, triazolyl, furylmethyl, thienylmethyl and pyridinylmethyl, any of which groups may be substituted by one or more substituents. Typical values of R¹ include *n*-butyl, isobutyl, 3-methylbutyl, *n*-pentyl, cyclohexyl, cyclohexylmethyl, phenyl, benzyl, phenylethyl, phenylpropyl, furylmethyl, thienylmethyl and pyridinylmethyl, any of which groups may be optionally substituted by one or more substituents.

Specific illustrations of particular substituents on the group R¹ include methyl, ethyl, *n*-propyl, phenyl, fluoro, trifluoromethyl, chloro, trichloromethyl, bromo, tribromomethyl, iodo, cyano, cyanomethyl, hydroxy, hydroxymethyl, methoxy, ethoxy and trifluoromethoxy.

More specific illustrations of particular substituents on the group R¹ include methyl, phenyl, fluoro, chloro, bromo, fluoro, iodo, trifluoromethyl, hydroxy, methoxy, ethoxy and trifluoromethoxy.

Representative values of R¹ include n-butyl, isobutyl, 3-methylbutyl, *n*-pentyl, cyclohexyl, cyclohexylmethyl, phenyl, benzyl, methylbenzyl, phenylbenzyl, fluorobenzyl, difluorobenzyl, chlorobenzyl, dichlorobenzyl, bromobenzyl, iodobenzyl, trifluoromethylbenzyl, hydroxybenzyl, methoxybenzyl, ethoxybenzyl, trifluoromethoxybenzyl, phenylethyl, phenylpropyl, furylmethyl, thienylmethyl and pyridinylmethyl.

Typical values of R² include cyclohexyl, phenyl and furyl, any of which groups may be optionally substituted by one or more substituents.

Specific illustrations of particular substituents on the group R² include methyl, ethyl, fluoro, chloro, bromo, cyano, hydros, methoxy and ethoxy.

More specific illustrations of particular substituents on the group R² include fluoro, chloro and methoxy.

Representative values of R² include cyclohexyl, phenyl, fluorophenyl, difluorophenyl, chlorophenyl, methoxyphenyl and furyl. Preferred values of R² include phenyl, fluorophenyl and difluorophenyl.

Suitable values of R³ include hydrogen and methyl, preferably hydrogen.

A particular sub-class of compounds according to the invention is represented by the compounds of formula IIA, and pharmaceutically acceptable salts thereof: 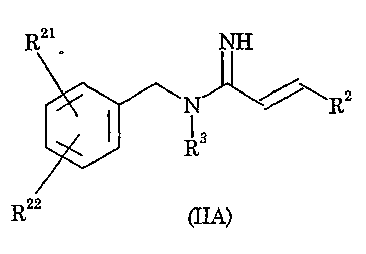 wherein R² and R³ are as defined above;

R²¹ represents hydrogen, C₁₋₆ alkyl, aryl, halogen, trihalo(C₁₋₆)alkyl, hydroxy, C₁₋₆ alkoxy or trihalo(C₁₋₆)alkoxy; and

R²² represents hydrogen or halogen.

Particular values of R²¹ include hydrogen, methyl, phenyl, fluoro, chloro, bromo, iodo, trifluoromethyl, hydroxy, methoxy, ethoxy and trifluoromethoxy.

Suitably, R²² represents hydrogen or chloro, especially hydrogen.

Another sub-class of compounds according to the invention is represented by the compounds of formula IIB, and pharmaceutically acceptable salts thereof: 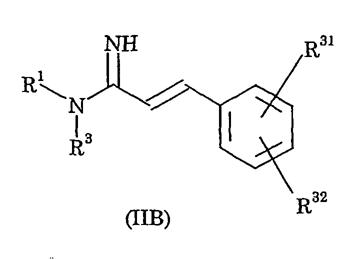 wherein R¹ and R³ are as defined above;

R³¹ represents hydrogen, C₁₋₆alkyl, halogen or C₁₋₆alkoxy; and

R³² represents hydrogen or halogen.

Particular values of R³¹ include hydrogen, fluoro, chloro and methoxy, especially fluoro.

Suitably, R³² represents hydrogen or fluoro, especially hydrogen.

A further sub-class of compounds according to the invention is represented by the compounds of formula IIC, and pharmaceutically acceptable salts, hydrates or prodrugs thereof: 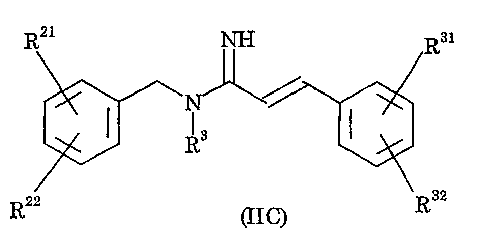 wherein R³, R²¹, R²², R³¹ and R³² are as defined above.

Another sub-class of compounds according to the invention is represented by the compounds of formula IID, and pharmaceutically acceptable salts, hydrates or prodrugs thereof: 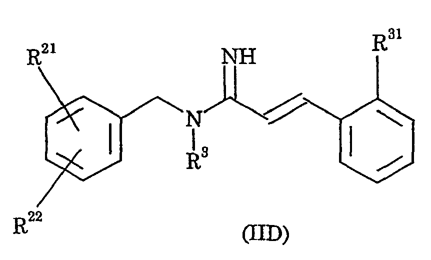 wherein R³, R²¹, R²² and R³¹ are as defined above.

Specific compounds within the scope of the present invention include:
(*E*)-N-(3-iodobenzyl)cinnamamidine;
(*E*)-N-(benzyl)cinnamamidine;
(*E*)-N-(4-chlorobenzyl)cinnamamidine;
(*E*)-N-(2-chlorobenzyl)cinnamamidine;
(*E*)-N-(3-chlorobenzyl)cinnamamidine;
(*E*)-N-(4-fluorobenzyl)cinnamamidine;
(*E*)-N-(4-trifluoromethylbenzyl)cinnamamidine;
(*E*)-N-(4-methoxybenzyl)cinnamamidine;
(*E*)-N-(4-methylbenzyl)cinnamamidine;
(*E*)-N-(3-methylbenzyl)cinnamamidine;
(*E*)-N-(2-methylbenzyl)cinnamamidine;
(*E*)-N-(3,4-dichlorobenzyl)cinnamamidine;
(*E*)-N-(2-phenylethyl)cinnamamidine;
(*E*)-N-(*R*)-α-methylbenzylcinnamamidine;
(*E*)-N-(*S*)-α-methylbenzylcinnamamidine;
(*E*)-N-(3-trifluoromethylbenzyl)cinnamamidine;
(*E*)-N-(3,5-dichlorobenzyl)cinnamamidine;
(*E*)-N-(2-methoxybenzyl)cinnamamidine;
(*E*)-N-(3-methoxybenzyl)cinnamamidine;
(*E*)-N-(2-trifluoromethylbenzyl)cinnamamidine;
(*E*)-N-(2,5-dichlorobenzyl)cinnamamidine;
(*E*)-N-(3-bromobenzyl)cinnamamidine;
(*E*)-N-(4-pyridylmethyl)cinnamamidine;
(*E*)-N-methyl-N-benzylcinnamamidine;
(*E*)-N-(2,3-dichlorobenzyl)cinnamamidine;
(*E*)-N-(cyclohexylmethyl)cinnamamidine;
(*E*)-N-(isobutyl)cinnamamidine;
(*E*)-N-(*n*-butyl)cinnamamidine;
(*E*)-N-(2-trifluoromethoxybenzyl)cinnamamidine;
(*E*)-N-(3-thienylmethyl)cinnamamidine;
(*E*)-N-(2-bromobenzyl)cinnamamidine;
(*E*)-N-(2-ethoxybenzyl)cinnamamidine;
(*E*)-N-(2-phenylbenzyl)cinnamamidine;
(*E*)-N-(2-furylmethyl)cinnamamidine;
(*E*)-N-(cyclohexyl)cinnamamidine;
(*E*)-N-(3-methyl-1-butyl)cinnamamidine;
(*E*)-2-(3-phenyl-1-imino-2-propenyl)-1,2,3,4-tetrahydroisoquinoline;
(*E*)-N-(*n*-pentyl)cinnamamidine;
(*E*)-N-(3-phenyl-1-propyl)cinnamamidine;
(*E*)-N-(2-hydroxybenzyl)cinnamamidine;
(*E*)-N-phenylcinnamamidine;
(*E*)-N-benzyl-2-chlorocinnamamidine;
(*E*)-N-benzyl-3-chlorocinnamamidine;
(*E*)-N-benzyl4-chlorocinnamamidine;
(*E*)-N-benzyl-4-fluorocinnamamidine;
(*E*)-N-benzyl-4-methoxycinnamamidine;
(*E*)-N-benzyl-3-methoxycinnamamidine;
(*E*)-N-benzyl-2-methoxycinnamamidine;
(*E*)-N-benzyl-3,4-difluorocinnamamidine;
(*E*)-N-benzyl-3-cyclohexaneacrylamidine;
and pharmaceutically acceptable salts, hydrates and prodrugs thereof.

The novel compounds of formula I, and the pharmaceutically acceptable salts and hydrates thereof, are useful for the relief of neurological and neurodegenerative disorders, including pain (and in particular neuropathic pain and headache, specifically migraine), epilepsy, stroke, anxiety, cerebral ischemia, muscular spasms, Alzheimer's Disease, Huntington's Disease and Parkinson's Disease.

For the treatment of any of these neurological and neurodegenerative diseases, a compound of formula I is administered in an amount that is effective to treat or prevent the said disease or condition. The compound may be administered orally, topically, parenterally, by inhalation spray or rectally, in dosages containing conventional non-toxic pharmaceutically acceptable diluents, adjuvants and vehicles. The term "parenteral" as used herein includes subcutaneous, intravenous, intramuscular, intradermal, epidural, and intrasternal injection or infusion techniques.

The therapeutic dose of the compound of formula I will vary with the nature or severity of the condition to be treated, the particular compound selected, its route of administration and other factors. It will also vary according to the age, weight and response of the individual patient. A representative dose ranges from about 0.001 mg/kg per day to about 100 mg/kg per day.

In another aspect, the present invention provides a pharmaceutical composition comprising a novel compound of formula I or a pharmaceutically acceptable salt or hydrate thereof in combination with a pharmaceutically acceptable carrier. Optionally other therapeutic ingredients may be included as well. Examples of dosage forms include tablets, troches, dispersions, suspensions, solutions, capsules, creams, ointments and aerosols.

The compositions may be presented in multiple dosage containers or in unit dosage form and prepared by methods well-known in the art of pharmacy.

The compounds of formula I can be combined as the active ingredient with the pharmaceutical carrier according to conventional pharmaceutical compounding techniques. The carrier may take a wide variety of forms depending on the form desired. In preparing oral dosage forms, any of the usual pharmaceutical media may be employed, such as, for example, water, alcohols, oils, flavouring agents, preservatives and colouring agents in the case of oral liquid preparations such as, for example, suspensions, elixirs and solutions; or starches, sugars, microcrystalline cellulose, diluents, granulating agents, lubricants, binders and disintegrating agents in the case of oral solid preparations such as, for example, powders, capsules and tablets. Solid oral preparations are preferred over liquid preparations. If desired, tablets may be coated by standard aqueous or nonaqueous techniques.

Examples of suitable dosage units typically range from about 0.01 mg to about 1.0 g of the active ingredient.

The diseases or conditions described herein may be effectively treated by the administration of from about 0.01 to about 50 mg of the compound per kilogram of body weight per day, or alternatively about 0.5 mg to about 3.5 g per patient per day.

The active ingredient may be combined with the carrier materials to produce the dosage form. For example, a formulation intended for oral administration to humans may contain from about 0.5 mg to about 5 g of the compound, compounded with an appropriate and convenient amount of carrier material which may vary from about 5 to about 95 percent of the total composition. Dosage units will generally contain between from about 1 mg to about 1000 mg of active ingredient, typically 25 mg, 50 mg, 100 mg, 200 mg, 300 mg, 400 mg, 500 mg, 600 mg, 800 mg or 1000 mg.

It will be understood that the specific dose level for any particular patient depends upon a variety of factors including the age, body weight, general health, sex, diet, time of administration, route of administration, rate of excretion, drug combination and the severity of the particular disease or condition undergoing therapy.

The compounds of formula I as defined above may be prepared by a process which comprises reacting a compound of formula III with a compound of formula IV: 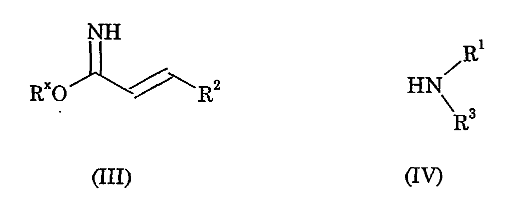 wherein R¹, R² and R³ are as defined above, and R^{x} represents C₁₋₆ alkyl, preferably methyl or ethyl.

The reaction between compounds III and IV is conveniently effected by stirring the reactants in a suitable solvent, typically methanol.

The intermediates of formula III may be prepared by reacting a compound of formula V with a compound of formula VI: 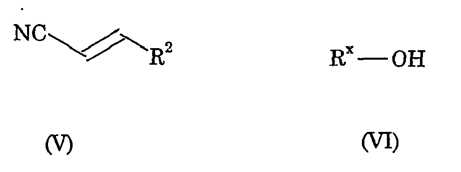 wherein R² and R^{x} are as defined above.

The reaction between the compounds of formula V and VI is conveniently effected by stirring the reactants in the presence of hydrogen chloride, in a suitable solvent, typically ether.

In another procedure, the compounds of formula I as described above may be prepared by a process which comprises reacting a compound of formula IV as defined above with a compound of formula VII: 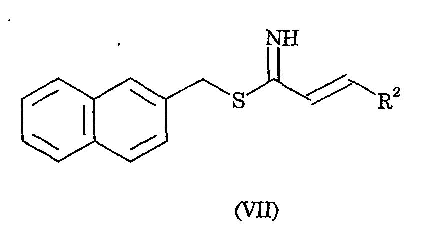 wherein R² is as defined above.

The reaction between compounds IV and VII is conveniently effected by stirring the reactants in a suitable solvent, typically chloroform or ethanol, at room temperature.

The intermediates of formula VII above may be prepared by reacting a compound of formula VIII with a compound of formula IX: 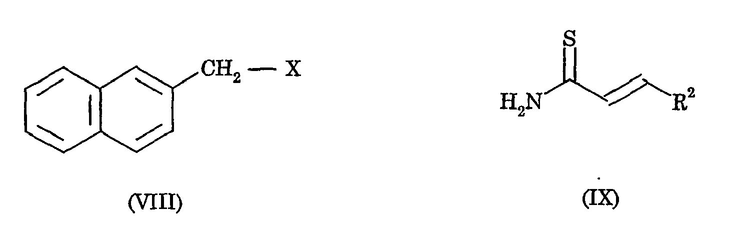 wherein R² is as defined above, and X represents a halogen atom, e.g. bromo.

The reaction between compounds VIII and IX is conveniently effected by refluxing the reactants in a suitable solvent, typically chloroform.

The intermediates of formula IX above may be prepared by reacting the compound of formula X with a compound of formula XI: 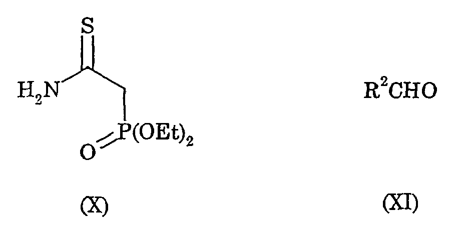 wherein R² is as defined above.

The reaction between compounds X and XI is conveniently effected by treating compound X with a strong base, e.g. sodium hydride, in the presence of a suitable solvent, typically THF, then adding compound XI and stirring the reaction mixture at a temperature between 0°C and room temperature.

In a further procedure, the compounds of formula I as defined above may be prepared by a process which comprises reacting a compound of formula XI as defined above with a compound of formula XII: 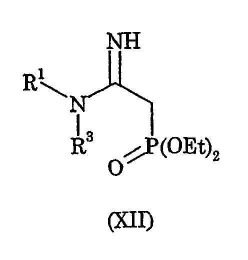 wherein R¹ and R³ are as defined above.

The reaction between compounds XI and XII is conveniently effected under basic conditions. For example, the reactants may be stirred with sodium hydroxide at room temperature in a suitable solvent, e.g. dichloromethane, typically in the presence of tetra-*n*-butylammonium bromide; or the reactants may be heated with potassium carbonate at reflux in a suitable solvent, typically tetrahydrofuran.

The intermediates of formula XII above may be prepared by reacting a compound of formula IV as defined above with a compound of formula XIII: 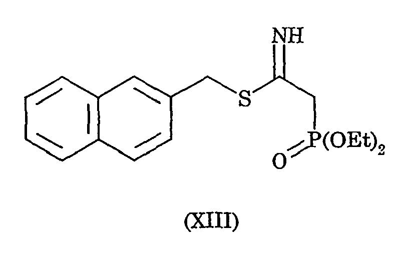 under conditions analogous to those described above for the reaction between compounds IV and VII.

The intermediates of formula XIII above may be prepared by reacting a compound of formula VIII with a compound of formula X, under conditions analogous to those described above for the reaction between compounds VIII and IX.

Where they are not commercially available, the starting materials of formula IV, V, VI, VIII, X and XI may be prepared by methods analogous to those described in the accompanying Examples, or by standard methods well known from the art.

It will be understood that any compound of formula I initially obtained from any of the above processes may, where appropriate, subsequently be elaborated into a further compound of formula I by techniques known from the art.

Where the above-described processes for the preparation of the compounds according to the invention give rise to mixtures of stereoisomers, these isomers may be separated by conventional techniques such as preparative chromatography or crystallisation. The novel compounds may be prepared in racemic form, or individual enantiomers may be prepared either by enantiospecific synthesis or by resolution. The novel compounds may, for example, be resolved into their component enantiomers by standard techniques such as preparative HPLC, or the formation of diastereomeric pairs by salt formation with an optically active acid, such as (-)-di-*p*-toluoyl-d-tartaric acid and/or (+)-di-*p*-toluoyl-1-tartaric acid, followed by fractional crystallization and regeneration of the free base. The novel compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The following Examples illustrate the preparation of compounds according to the invention.

The following assays are used to demonstrate biological activity and utility for the compounds of formula I.

### [³H]-Ifenprodil binding to recombinant human NR1a/NR2B receptors

Ifenprodil is an NMDA receptor antagonist which acts through a distinct modulatory site from those of glutamate, glycine and MK-801 (dizocilpine) and is selective for NR2B-containing receptors (Grimwood et al., J. Neurochem., 1996, 66, 2589-2595). [³H]-Ifenprodil binding to cell membranes expressing recombinant human NR1a/NR2B receptors is determined essentially as described by Grimwood et al., NeuroReport, 1999, 10, 461-465.

The compounds of the accompanying Examples were tested in the above assay, and all were found to demonstrate a 50% inhibition of [³H]-ifenprodil binding at a concentration of 5 µM or less.

### Functional Ca⁺⁺ antagonism assay-FLIPR

Human NR1a/2B receptor transfected cells are plated in a 96-well format and grown for one day in normal growth media (Dulbeccos MEM with Na pyruvate). NR1a/2B expression in these cells is induced by the addition of dexamethasone in the presence of ketamine for 16-24 hours. After receptor induction cells are washed with assay buffer (Hanks balanced salt solution (HBSS-Mg free) containing 20 mM HEPES, 0.1% BSA, 2 mM CaCl₂ and 250 µM probenecid). Each 96 well cell plate is loaded with the Ca⁺⁺ sensitive dye Fluo-3 (Molecular Probes, Inc.) in assay buffer. The cells are then washed with assay buffer leaving them in 100 µl buffer. Test compounds in solution are pipetted by FLIPR (Fluorometric Imaging Plate Reader, Molecular Dynamics) for 2 min pretreatment. During this time the fluorescence intensity is recorded (excitation at 488 nm and emission at 530 nm). The glutamate/glycine 50 µl agonist solution (final concentration 1 µM/1 µM) is then added by FLIPR into each well already containing 150 µl of buffer (containing the test compound or vehicle) and the fluorescence is continuously monitored for 10 min. Fluorescence values in the presence of an antagonist are compared to those for the agonist alone.

Of those compounds of the accompanying Examples which were tested in this assay, all were found to possess an EC₅₀ value of 10 µM or less.

### Carrageenan-induced mechanical hyperalgesia in rats

The ability of test compounds to reverse carrageenan induced hyperalgesia can be determined using the method described by Boyce et al., Neuropharmacology, 1994, 33, 1609-1611.

### EXAMPLE 1

### (E)-N-(3-Iodobenzyl)cinnamamidine hydrochloride

3-Iodobenzylamine (85µl, 0.64mmol) was added to a solution of *(E)-*ethyl cinnamimidate hydrochloride (112.9mg, 0.571mmol) [prepared by the method of E.C. Roberts and Y.F. Shealy, J. Het. Chem. 1974, 11, 547] in methanol (1ml) and the mixture stirred at room temperature overnight (18 hours). The mixture was diluted with diethyl ether (3ml), the crystalline product collected under suction, washed with methanol-diethyl ether (1:3) then diethyl ether and dried *in vacuo* to give the title product as a white solid (173mg, 76%); δ_{H} (360MHz, DMSO-d₆) 4.60 (2H, d, J 5.2Hz, ArCH₂N), 6.89 (1H, d, J 16.5Hz, CH=C*H*Ph), 7.22 (1H, t, J 7.8Hz, ArH), 7.46 (1H, d, J 7.8Hz, ArH), 7.50 (3H, m, ArH), 7.60 (2H, m, ArH), 7.73 (1H, d, J 7.9Hz, ArH), 7.83 (1H, s, ArH), 7.92 (1H, d, J 16.5Hz, C*H*=CHPh), 9.03 (1H, br s, NH), 9.52 (1H, br s, NH), 10.26 (1H, br s, NH); m/z (ES⁺) 363 ([M+H]⁺).

The following Examples were prepared in an analogous process to the one described above:

### EXAMPLE 2

### (E)-N-(Benzyl)cinnamamidine hydrochloride

(Found: C, 70.35; H, 6.16; N, 10.30. C₁₆H₁₇ClN₂ requires C, 70.45; H, 6.28; N, 10.27%); δ_{H} (360MHz, DMSO-d₆) 4.64 (2H, s, PhCH₂N), 6.93 (1H, d, J 16.5Hz, CH=C*H*Ph), 7.37 (1H, m, ArH), 7.42 (4H, m, ArH), 7.49 (3H, m, ArH), 7.60 (2H, m, ArH), 7.96 (1H, d, J 16.5Hz, C*H*=CHPh), 9.04 (1H, br s, NH), 9.54 (1H, br s, NH), 10.31 (1H, br s, NH); m/z (ES⁺) 237 ([M+H]⁺).

### EXAMPLE 3

### (E)-N-(4-Chlorobenzyl)cinnamamidine hydrochloride

δ_{H} (360MHz, DMSO-d₆) 4.63 (2H, s, ArCH₂N), 6.90 (1H, d, J 16.4Hz, CH=C*H*Ph), 7.48 (7H, m, ArH), 7.60 (2H, m, ArH), 7.93 (1H, d, J 16.4Hz, C*H*=CHPh), 9.04 (1H, br s, NH), 9.53 (1H, br s, NH), 10.30 (1H, br s, NH); m/z (ES⁺) 271/273 ([M+H]⁺).

### EXAMPLE 4

### (E)-N-(2-Chlorobenzyl)cinnamamidine hydrochloride

δ_{H} (360MHz, DMSO-d₆) 4.68 (2H, s, ArCH₂N), 6.91 (1H, d, J 16.5Hz, CH=C*H*Ph), 7.42-7.62 (9H, m, ArH), 7.94 (1H, d, J 16.5Hz, C*H*=CHPh), 9.08 (1H, br s, NH), 9.58 (1H, br s, NH), 10.10 (1H, br s, NH); m/z (ES⁺) 271/273 ([M+H]⁺).

### EXAMPLE 5

### (E)-N-(3-Chlorobenzyl)cinnamamidine hydrochloride

δ_{H} (360MHz, DMSO-d₆) 4.65 (2H, s, PhCH₂N), 6.91(1H, d, J 16.5Hz, CH=C*H*Ph), 7.47 (7H, m, ArH), 7.60 (2H, m, ArH), 7.95 (1H, d, J 16.5Hz, C*H*=CHPh), 9.07 (1H, br s, NH), 9.55(1H, br s, NH), 10.35 (1H, br s, NH); m/z (ES⁺) 271/273 ([M+H]⁺).

### EXAMPLE 6

### (E)-N-(4-Fluorobenzyl)cinnamamidine hydrochloride

δ_{H} (360MHz, DMSO-d₆) 4.61 (2H, s, ArCH₂N), 6.89 (1H, d, J 16.4Hz, CH=C*H*Ph), 7.26 (2H, m, ArH), 7.50 (5H, m, ArH), 7.59 (2H, m, ArH), 7.92 (1H, d, J 16.5Hz, C*H*=CHPh), 9.03 (1H, br s, NH), 9.51 (1H, br s, NH), 10.26 (1H, br s, NH); m/z (ES⁺) 255 ([M+H]⁺).

### EXAMPLE 7

### (E)-N-(4-Trifluoromethylbenzyl)cinnamamidine hydrochloride

δ_{H} (360MHz, DMSO-d₆) 4.75 (2H, s, ArCH₂N), 6.91 (1H, d, J 16.5Hz, CH=C*H*Ph), 7.51 (3H, m, ArH), 7.63 (4H, m, ArH), 7.80 (2H, m, ArH), 7.94 (1H, d, J 16.5Hz, C*H*=CHPh); m/z (ES⁺) 305 ([M+H]⁺).

### EXAMPLE 8

### (E)-N-(4-Methoxybenzyl)cinnamamidine hydrochloride

δ_{H} (360MHz, DMSO-d₆) 3.76 (3H, s, OCH₃), 4.54 (2H, s, ArCH₂N), 6.88 (1H, d, J 16.5Hz, CH=C*H*Ph), 6.98 (2H, m, ArH), 7.37 (2H, m, ArH), 7.49 (3H, m, ArH), 7.59 (2H, m, ArH), 7.92 (1H, d, J 16.5Hz, C*H*=CHPh); m/z (ES⁺) 267((M+H]⁺).

### EXAMPLE 9

### (E)-N-(4-Methylbenzyl)cinnamamidine hydrochloride

δ_{H} (360MHz, DMSO-d₆) 2.31 (3H, s, ArCH₃), 4.57 (2H, s, ArCH₂N), 6.91(1H, d, J 16.5Hz, CH=C*H*Ph), 7.22 (2H, m, ArH), 7.35 (2H, m, ArH), 7.50 (3H, m, ArH), 7.59 (2H, m, ArH), 7.94 (1H, d, J 16.5Hz, C*H*=CHPh), 8.99 (1H, br s, NH), 9.51 (1H, br s, NH), 10.24 (1H, br s, NH); m/z (ES⁺) 251([M+H]⁺).

### EXAMPLE 10

### (E)-N-(3-Methylbenzyl)cinnamamidine hydrochloride

δ_{H} (360MHz, DMSO-d₆) 2.33 (3H, s, ArCH₃), 4.58 (2H, s, ArCH₂N), 6.91(1H, d, J 16.4Hz, CH=C*H*Ph), 7.15-7.32 (4H, m, ArH), 7.49 (3H, m, ArH), 7.60 (2H, m, ArH), 7.91(1H, d, J 16.5Hz, C*H*=CHPh); m/z (ES⁺) 251([M+H]⁺).

### EXAMPLE 11

### (E)-N-(2-Methylbenzyl)cinnamamidine hydrochloride

δ_{H} (360MHz, DMSO-d₆) 2.33 (3H, s, ArCH₃), 4.60 (2H, s, ArCH₂N), 6.99 (1H, d, J 16.5Hz, CH=C*H*Ph), 7.26 (4H, m, ArH), 7.50 (3H, m, ArH), 7.60 (2H, m, ArH), 8:00 (1H, d, J 16.5Hz, C*H*=CHPh); m/z (ES⁺) 251 ([M+H]⁺).

### EXAMPLE 12

### (E)-N-(3,4-Dichlorobenzyl)cinnamamidine hydrochloride

δ_{H} (360MHz, DMSO-d₆) 4.66 (2H, s, ArCH₂N), 6.94 (1H, d, J 16.5Hz, CH=C*H*Ph), 7.47 (4H, m, ArH), 7.60 (2H, m, ArH), 7.69 (1H, m, ArH), 7.77 (1H, m, ArH), 7.98 (1H, d, J 16.5Hz, C*H*=CHPh), 9.14 (1H, br s, NH), 9.61 (1H, br s, NH), 10.48 (1H, br s, NH); m/z (ES⁺) 305/307([M+H]⁺).

### EXAMPLE 13

### (E)-N-(2-Phemylethyl)cinnamamidine hydrochloride

δ_{H} (360MHz, DMSO-d₆) 2.95 (2H, t, NCH₂C*H*₂Ph), 3.61 (2H, t, NC*H*₂CH₂Ph), 6.86 (1H, d, J 16.5Hz, CH=C*H*Ph), 7.34 (5H, m, ArH), 7.50 (3H, m, ArH), 7.58 (2H, m, ArH), 7.86 (1H, d, J 16.5Hz, C*H*=CHPh), 8.85 (1H, br s, NH), 9.37 (1H, br s, NH), 9.94 (1H, br s, NH); m/z (ES⁺) 251([M+H]⁺).

### EXAMPLE 14

### (E)-N-(R)-α-Methylbenzylcinnamamidine hydrochloride

δ_{H} (360MHz, DMSO-d₆) 1.57 (3H, d, J 6.7Hz, NCH(C*H*₃)Ph), 5.11 (1H, m, NC*H*(CH₃)Ph), 6.96 (1H, d, J 16.5Hz, CH=C*H*Ph), 7.31-7.49 (8H, m, ArH), 7.61 (2H, m, ArH), 7.91 (1H, d, J 16.5Hz, C*H*=CHPh); m/z (ES⁺) 251([M+H]⁺).

### EXAMPLE 15

### (E)-N-(S)-α-Methylbenzylcinnamamidine hydrochloride

δ_{H} (360MHz, DMSO-d₆) 1.57 (3H, d, J 6.7Hz, NCH(C*H*₃)Ph), 5.17 (1H, m, NC*H*(CH₃)Ph), 7.05 (1H, d, J 16.5Hz, CH=C*H*Ph), 7.30-7.52 (8H, m, ArH), 7.60 (2H, m, ArH), 7.99 (1H, d, J 16.5Hz, C*H*=CHPh); m/z (ES⁺) 251([M+H]⁺).

### EXAMPLE 16

### (E)-N-(3-Trifluoromethylbenzyl)cinnamamidine hydrochloride

δ_{H} (360MHz, DMSO-d₆) 4.75 (2H, s, ArCH₂N), 6.93 (1H, d, J 16.5Hz, CH=C*H*Ph), 7.50 (3H, m, ArH), 7.59-7.77 (5H, m, ArH), 7.85 (1H, m, ArH), 7.97 (1H, d, J 16.5Hz, C*H*=CHPh), 9.13 (1H, br s, NH), 9.60 (1H, br s, NH), 10.43 (1H, br s, NH); m/z (ES⁺) 305 ([M+H]⁺).

### EXAMPLE 17

### (E)-N-(3,5-Dichlorobenzyl)cinnamamidine hydrochloride

δ_{H} (360MHz, DMSO-d₆) 4.67 (2H, s, ArCH₂N), 6.92 (1H, d, J 16.5Hz, CH=C*H*Ph), 7.50 (3H, m, ArH), 7.55 (2H, m, ArH), 7.60 (3H, m, ArH), 7.96 (1H, d, J 16.5Hz, C*H*=CHPh), 9.13 (1H, br s, NH), 9.60 (1H, br s, NH), 10.44 (1H, br s, NH); m/z (ES⁺) 305/307([M+H]⁺).

### EXAMPLE 18

### (E)-N-(2-Methoxvbenzyl)cinnamamidine hydrochloride

δ_{H} (360MHz, DMSO-d₆) 3.85 (3H, s, ArOC*H*₃), 4.54 (2H, s, ArCH₂N), 6.92 (1H, d, J 16.5Hz, CH=C*H*Ph), 6.99 (1H, m, ArH), 7.09 (1H, d, ArH), 7.31-7.39 (2H, m, ArH), 7.49 (3H, m, ArH), 7.59 (2H, m, ArH), 7.94 (1H, d, J 16.5Hz, C*H*=CHPh), 8.91 (1H, br s, NH), 9.53 (1H, br s, NH), 9.96 (1H, br s, NH); m/z (ES⁺) 267([M+H]⁺).

### EXAMPLE 19

### (E)-N-(3-Methoxybenzyl)cinnamamidine hydrochloride

δ_{H} (360MHz, DMSO-d₆) 3.77 (3H, s, ArOC*H*₃), 4.59 (2H, d; ArCH₂N), 6.91-7.04 (4H, m, ArH, CH=C*H*Ph), 7.33 (1H, m, ArH), 7.49 (3H, m, ArH), 7.59 (2H, m, ArH), 7.96 (1H, d, J 16.5Hz, C*H*=CHPh), 9.03 (1H, br s, NH), 9.54 (1H, br s, NH), 10.33. (1H, br s, NH); m/z (ES⁺) 267([M+H]⁺).

### EXAMPLE 20

### (E)-N-(2-Trifluoromethylbenzyl)cinnamamidine hydrochloride

δ_{H} (360MHz, DMSO-d₆) 4.77 (2H, s, ArCH₂N), 6.93 (1H, d, J 16.5Hz, CH=C*H*Ph), 7.50 (3H, m, ArH), 7.62 (4H, m, ArH), 7.77 (1H, m, ArH),7.84 (1H, m, ArH), 7.96 (1H, d, J 16.5Hz, C*H*=CHPh), 9.18 (1H, br s, NH), 9.68 (1H, br s, NH), 10.12 (1H, br s, NH); m/z (ES⁺) 305 ([M+H]⁺).

### EXAMPLE 21

### (E)-N-(2,5-Dichlorobenzyl)cinnamamidine hydrochloride

δ_{H} (360MHz, DMSO-d₆) 4.68 (2H, s, ArCH₂N), 6.98 (1H, d, J 16.1Hz, CH=C*H*Ph), 7.50-7.59 (8H, m, ArH), 8.01 (1H, d, J 16.6Hz, C*H*=CHPh); m/z (ES⁺) 305/307([M+H]⁺).

### EXAMPLE 22

### (E)-N-(3-Bromobenzyl)cinnamamidine hydrochloride

δ_{H} (360MHz, DMSO-d₆) 4.64 (2H, d, ArCH₂N), 6.92 (1H, d, J 16.5Hz, CH=C*H*Ph), 7.28-7.67 (9H, m, ArH), 7.96 (1H, d, J 16.5Hz, C*H*=CHPh), 9.08 (1H, br s, NH), 9.57 (1H, br s, NH), 10.38 (1H, br s, NH); m/z (ES⁺) 315/317 ([M+H]⁺).

### EXAMPLE 23

### (E)-N-(4-Pyridylmethyl)cinnamamidine hydrochloride

δ_{H} (360MHz, DMSO-d₆) 4.99 (2H, d, ArCH₂N), 7.03 (1H, d; J 16.4Hz, CH=C*H*Ph), 7.52 (3H, m, ArH), 7.62 (2H, m, ArH), 7.95 (2H, m, ArH), 8.08 (1H, d, J 16.4Hz, C*H*=CHPh), 8.88 (2H, d, ArH), 9.38 (1H, br s, NH), 9.78 (1H, br s, NH), 10.86 (1H, br s, NH); m/z (ES⁺) 238 ([M+H]⁺).

### EXAMPLE 24

### (E)-N-Methyl-N-benzylcinnamamidine hydrochloride

δ_{H} (400MHz, DMSO-d₆, 340K) 3.11 (3H, s, NC*H*₃), 4.93 (2H, s, ArCH₂N), 7.17 (1H, d, J 16.0 Hz, CH=C*H*Ph), 7.32-7.48 (8H, m, ArH), 7.61-7.72 (3H, m, ArH, C*H*=CHPh); m/z (ES⁺) 251 ([M+H]⁺).

### EXAMPLE 25

### (E)-N-(2,3-Dichlorobenzyl)cinnamamidine hydrochloride

δ_{H} (360MHz, DMSO-d₆) 4.72 (2H, s, ArCH₂N), 6.97 (1H, d, J 16.4Hz, CH=C*H*Ph), 7.44 (2H, m, ArH), 7.50 (3H, m, ArH), 7.61 (2H, m, ArH), 7.68 (1H, m, ArH), 8.00 (1H, d, J 16.5Hz, C*H*=CHPh), 9.67 (3H, br s, 3 x NH); m/z (ES⁺) 305/307([M+H]⁺).

### EXAMPLE 26

### (E)-N-(Cyclohexylmethyl)cinnamamidine hydrochloride

δ_{H} (360MHz, DMSO-d₆) 0.95-1.02 (2H, m), 1.18-1.24 (3H, m), 1.65-1.79 (6H, m), 3.20 (2H, t, CH₂N), 6.92 (1H, d, J 16.4Hz, CH=C*H*Ph), 7.49 (3H, m, ArH), 7.59 (2H, m, ArH), 7.88 (1H, d, J 16.5Hz, C*H*=CHPh), 8.76 (1H, br s, NH), 9.32 (1H, br s, NH), 9.83 (1H, br s, NH); m/z (ES⁺) 243([M+H]⁺).

### EXAMPLE 27

### (E)-N-(Isobutyl)cinnamamidine hydrochloride

δ_{H} (360MHz, DMSO-d₆) 0.95 (6H, m), 1.95 (1H, m), 3.18 (2H, d, CH₂N), 6.98 (1H, d, J 16.5Hz, CH=C*H*Ph), 7.49 (3H, m, ArH), 7.59 (2H, m, ArH), 7.92 (1H, d, J 16.5Hz, C*H*=CHPh); m/z (ES⁺) 203([M+H]⁺).

### EXAMPLE 28

### (E)-N-(n-Bulyl)cinnamamidine hydrochloride

δ_{H} (360MHz, DMSO-d₆) 0.92 (3H, m), 1.39 (2H, m), 1.59 (2H, m), 3.32 (2H, s, CH₂N), 6.90 (1H, d, J 16.5Hz, CH=C*H*Ph), 7.49 (3H, m, ArH), 7.58 (2H, m, ArH), 7.89 (1H, d, J 16.5Hz, C*H*=CHPh), 8.76 (1H, br s, NH), 9.34 (1H, br s, NH), 9.88 (1H, br s, NH); m/z (ES⁺) 203([M+H]⁺).

### EXAMPLE 29

### (E)-N-(2-Trifluoromethoxybenzyl)cinnamamidine hydrochloride

δ_{H} (360MHz, DMSO-d₆) 4.69 (2H, d, ArCH₂N), 6.91 (1H, d, J 16.5Hz, CH=C*H*Ph), 7.45-7.62 (9H, m, ArH), 7.94 (1H, d, J 16.5Hz, C*H*=CHPh), 9.14 (1H, br s, NH), 9.63 (1H, br s, NH), 10.11 (1H, br s, NH); m/z (ES⁺) 321 ([M+H]⁺).

### EXAMPLE 30

### (E)-N-(3-Thienylmethyl)cinnamamidine hydrochloride

δ_{H} (360MHz, DMSO-d₆) 4.83 (2H, d, ArCH₂N), 6.84 (1H, d, J 16.4Hz, CH=C*H*Ph), 7.07 (2H, m, ArH), 7.23 (2H, m, ArH), 7.56 (4H, m, ArH), 7.87 (1H, d, J 16.5Hz, C*H*=CHPh), 9.48 (1H, br s, NH), 9.50 (1H, br s, NH), 10.22 (1H, br s, NH); m/z (ES⁺) 243 ([M+H]⁺).

### EXAMPLE 31

### (E)-N-(2-Bromobenzyl)cinnamamidine hydrochloride

δ_{H} (250MHz, DMSO-d₆) 4.64 (2H, s, ArCH₂N), 6.90 (1H, d, J 16.5Hz, CH=C*H*Ph), 7.36 (1H, m, ArH), 7.49 (5H, m, ArH), 7.60 (2H, m, ArH), 7.74 (1H, d, J 10.9Hz, ArH), 7.92 (1H, d, J 16.5Hz, C*H*=CHPh); m/z (ES⁺) 315/317 ([M+H]⁺).

### EXAMPLE 32

### (E)-N-(2-Ethoxybenzyl)cinnamamidine hydrochloride

δ_{H} (360MHz, DMSO-d₆) 1.35 (3H, t, CH₂C*H*₃), 4.09 (2H, q, C*H*₂CH₃), 4.54 (2H, s, ArCH₂N), 6.87 (1H, d, J 16.4Hz, C*H*=CHPh), 6.98 (1H, dt, ArH), 7.07 (1H, d, ArH), 7.33 (2H, m, ArH), 7.49 (3H, m, ArH), 7.58 (2H, m, ArH), 7.88 (1H, d, J 16.5Hz, C*H*=CHPh), 8.83 (1H, br s, NH), 9.42 (1H, br s, NH), 9.82 (1H, br s, NH); m/z (ES⁺) 281 ([M+H]⁺).

### EXAMPLE 33

### (E)-N-(2-Phenylbenzyl)cinnamamidine hydrochloride

δ_{H} (360MHz, DMSO-d₆) 4.47 (2H, s, ArCH₂N), 6.79 (1H, d, J 16.4Hz, CH=C*H*Ph), 7.34-7.46 (12H, m, ArH), 7.58 (2H, m, ArH), 7.77 (1H, d, J 16.4Hz, C*H*=CHPh), 8.80 (1H, br s, NH), 9.26 (1H, br s, NH), 9.83 (1H. br s, NH); m/z (ES⁺) 313 ([M+H]⁺).

### EXAMPLE 34

### (E)-N-(2-Furylmethyl)cinnamamidine hydrochloride

δ_{H} (360MHz, DMSO-d₆) 4.64 (2H, s, ArCH₂N), 6.49 (1H, m, ArH), 6.55 (1H, m, ArH), 6.80 (1H, d, J 16.4Hz, CH=C*H*Ph), 7.50 (2H, m, ArH), 7.59 (2H, m, ArH), 7,73 (1H, m, ArH), 7.83 (1H, d, J 16.5Hz, C*H*=CHPh); m/z (ES⁺) 226 ([M+H]⁺).

### EXAMPLE 35

### (E)-N-(Cyclohexyl)cinnamamidine hydrochloride

δ_{H} (360MHz, DMSO-d₆) 1.19 (1H, m, AlkH), 1.33 (4H, m, AlkH), 1.61 (1H, m, AlkH), 1.75 (2H, m, AlkH), 1.91 (2H, m, AlkH), 3.65 (1H, m, AlkH), 6.84 (1H, d, J 16.5Hz, CH=C*H*Ph), 7.50 (3H, m, ArH), 7.58 (2H, m, ArH), 7.83 (1H, d, J 16.5Hz, C*H*=CHPh), 8.69 (1H, br s, NH), 9.21 (1H, br s, NH), 9.49 (1H, br s, NH); m/z (ES⁺) 229 ([M+H]⁺),

### EXAMPLE 36

### (E)-N-(3-Methyl-1-butyl)cinnamamidine hydrochloride

δ_{H} (360MHz, MeOH-d₄) 1.00 (6H, m, AlkH), 1.62 (1H, m, AlkH), 3.39 (2H, t, AlkH), 3.83 (2H, s, AlkH), 6.70 (1H, d, J 16.5Hz, CH=C*H*Ph), 7.47 (2H, m, ArH), 7.54 (1H, m, ArH), 7.62 (2H, m, ArH), 7.70 (2H, d, J 16.4Hz, C*H*=CHPh); m/z (ES⁺) 217 ([M+H]⁺).

### EXAMPLE 37

### (E)-2-(3-Phenyl-1-imino-2-propenyl)-1,2,3,4-tetrahydroisoquinoline

δ_{H} (400MHz, DMSO-d₆, 333K) 3.01 (2H, m, AlkH), 3.70 (2H, br s, AlkH), 4.75 (2H, br s, AlkH), 7.20 (1H, d, J 16.1Hz, CH=C*H*Ph), 7.23-7.29 (4H, m, ArH), 7.50 (3H, m, ArH), 7.62 (1H, d, J 16.1Hz, C*H*=CHPh), 7.76 (2H, m, ArH), 8.98 (3H, br s, 3 x NH); m/z (ES⁺) 263 ([M+H]⁺).

### EXAMPLE 38

### (E)-N-(n-Pentyl)cinnamamidine hydrochloride

δ_{H} (360MHz, DMSO-d₆) 0.90 (3H, t, J 7.1Hz, CH₃), 1.33 (4H, m, AlkH), 1.61 (2H, m, AlkH), 6.85 (1H, d, J 16.5Hz, CH=C*H*Ph), 7.49 (3H, m, ArH), 7.59 (2H, m, ArH), 7.83 (1H, d, J 16.5Hz, C*H*=CHPh), 8.70 (1H, br s, NH), 9.30 (1H, br s, NH), 9.80 (1H, br s, NH); m/z (ES⁺) 217 ([M+H]⁺).

### EXAMPLE 39

### (E)-N-(3-Phenyl-1-propyl)cinnamamidine hydrogen oxalate

δ_{H} (360MHz, DMSO-d₆) 1.92 (2H, m, AlkH), 2.69 (2H, m, AlkH), 3.33 (2H, m, AlkH), 6.79 (1H, d, J 16.4Hz, CH=C*H*Ph), 7.26 (5H, m, ArH), 7.50 (3H, m, ArH), 7.60 (2H, m, ArH), 7.76 (1H, d, J 16.6Hz, C*H*=CHPh), 8.65 (1H, br s, NH), 9.21 (1H, br s, NH), 9.67 (1H, br s, NH); m/z (ES⁺) 265 ([M+H]⁺).

### EXAMPLE 40

### (E)-N-(2-Hydroxybenzyl)cinnamamidine

2-Hydroxybenzylamine (276.4mg, 2.24mmol) and sodium methoxide (123.2mg, 2.28mmol) were added to a solution of (*E*)-ethyl cinnamimidate hydrochloride (482.3mg, 2.28mmol) [prepared by the method of E.C. Roberts and Y.F. Shealy, *J. Het. Chem.* **1974**, *11*, 547] in methanol (10ml) and the mixture stirred at room temperature for 6.5 hours. The reaction mixture was then poured into water (50ml) and extracted with dichloromethane (50ml then 2 x 25ml). The combined extracts were dried (MgSO₄) and concentrated to a small volume. Methanol was added and the precipitate collected under suction, washed with methanol and dried *in vacuo* to give the title compound as a buff solid (90.2mg, 16%); δ_{H} (360MHz, DMSO-d₆) 4.25 (2H, s, ArCH₂N), 6.61 (1H, d, J 16.5Hz, CH=C*H*Ph), 6.67 (2H, m, ArH), 7.08 (2H, m, ArH), 7.31-7.43 (4H, m, ArH and C*H*=CHPh), 7.51 (2H, m, ArH); m/z (ES⁺) 253 ([M+H]⁺).

### EXAMPLE 41

### (E)-N-Phenylcinnamaraidine hydrochloride

### Step 1: (E)-S-(2-Naphthylmethyl)thiocinnamimidate hydrobromide

2-Bromomethylnaphthalene (371.2mg, 1.68mmol) was added to a suspension of thiocinnamamide (273.1mg, 1.67mmol) [prepared by the method of I.T. Barnish, C.W.G. Fishwick, D.R. Hill and C. Szantay, Jr., *Tetrahedron,* **1989,** *45,* 6771] in chloroform (5ml). The mixture was heated at reflux for 1.5 hours, allowed to cool and diluted with diethyl ether (5ml). The crystallised solid was collected under suction, washed with 50% chloroform in diethyl ether then diethyl ether and dried *in vacuo* to give the title compound as a pale yellow solid (436.6mg, 68%); δ_{H} (360MHz, CDCl₃) 5.11 (2H, s, ArCH₂S), 7.41-7.55 (6H, m, ArH), 7.66(2H, m, ArH), 7.76 (1H, d, J 16.2Hz, CH=C*H*Ph), 7.83-7.88 (3H, m, ArH), 7.96 (1H, d, J 16.2Hz, C*H*=CHPh), 8.02 (1H, s, ArH), 11.47 (1H, br s, NH), 12.28 (1H, br s, NH); m/z (ES⁺) 304 ([M+H]⁺), 141 (100%).

### Step 2: (E)-N-Phenylcinnamamidine hydrochloride

Aniline (51µl, 0.56mmol) was added to a solution of (*E*)-S-(2-naphthylmethyl)-thiocinnamimidate hydrobromide (216.1mg, 0.56mmol) in chloroform (4ml) and the mixture stirred at room temperature for 30 minutes. The reaction mixture was concentrated *in vacuo,* treated with saturated aqueous sodium hydrogen carbonate solution (20ml) and extracted with dichloromethane (2 x 10ml). The combined extracts were dried (MgSO₄), concentrated and the residue purified by flash chromatography eluting with dichloromethane-methanol-ammonia (180:8:1). The purified product free base (50.2mg) was dissolved in dichloromethane and treated with 1M HCl in diethyl ether (0.29ml). The mixture was evaporated and the residue recrystallised from methanol-diethyl ether to give the title compound (38.0mg, 26%); δ_{H} (360MHz, DMSO-d₆) 7.04 (1H, d, J 16.1Hz, CH=C*H*Ph), 7.41-7.47 (3H, m, ArH), 7.52-7.59 (5H, m, ArH), 7.65 (2H, m, ArH), 8.06 (1H, d, J 16.1Hz, C*H*=CHPh), 8.72 (1H, br s, NH), 9.67 (1H, br s, NH), 11.73 (1H, br s, NH); m/z (ES⁺) 223 ([M+H]⁺).

### EXAMPLE 42

### (E)-N-Benzyl-2-chlorocinnamamidine hydrochloride

### Step 1: 2-(Diethylphosphono)-S-(2-naphthylmethyl)thioacetimidate hydrobromide

2-Bromomethylnaphthalene (5.23g, 23.7mmol) was added to a solution of diethyl (2-amino-2-thioxoethyl)phosphonate (5.00g, 23.7mmol) in chloroform (50ml) The mixture was heated at reflux for 1.5 hours, allowed to cool and concentrated *in vacuo.* The residue was triturated with acetone and the resulting white solid collected under suction to give the title compound (7.84g, 77%); δ_{H} (360MHz, DMSO-d₆) 1.22 (6H, t, 7.0Hz, 2 x OCH₂C*H*₃) 3.81 (2H, d, J 22Hz, CH₂P), 4.09 (4H, m, 2 x OC*H*₂CH₃), 4.85 (2H, s, ArCH₂S), 7.56 (3H, m, ArH), 7.89-7.98 (3H, m, ArH), 8.04 (1H, s, ArH); m/z (ES⁺) 352 ([M+H]⁺):

### Step 2: N-Benzyl-2-(diethylphosphono)acetamidine hydrobromide

Benzylamine (0.54ml, 4.95mmol) was added to a suspension of 2-(diethylphosphono)-S-(2-naphthylmethyl)thioacetimidatehydrobromide (2.1187g, 4.90mmol) in ethanol (10ml). The mixture was stirred at room temperature for 2 hours, then warmed and diluted with diethyl ether to cloud point. It was allowed to cool to give a crystalline solid, which was collected under suction, washed with 10% ethanol in diethyl ether then diethyl ether and dried *in vacuo* to afford the title compound (0.8520g, 48%); δ_{H} (360MHz, DMSO-d₆) 1.24 (6H, t, 7.0Hz, 2 x OCH₂C*H*₃) 3.22 (2H, d, J 22Hz, CH₂P), 4.09 (4H, m, 2 x OC*H*₂CH₃), 4.53 (2H, s, ArCH₂N), 7.40 (5H, m, ArH); 9.04 (1H, v br s, NH), 9.24 (1H, v br s, NH), 9.94 (1H, v br s, NH); m/z (ES⁺) 285 ([M+H]⁺).

### Step 3: (E)-N-Benzyl-2-chlorocinnamamidine hydrochloride

A rapidly stirred mixture of N-benzyl-2-(diethylphosphono)acetamidine hydrobromide (196.0mg, 0.537mmol), 2-chlorobenzaldehyde (63µl, 0.56mmol) and potassium carbonate (324.2mg, 2.35mmol) in tetrahydrofuran (10ml) was heated at reflux for 6 hours. The reaction mixture was poured into water (50ml) and extracted with dichloromethane (2 x 25ml). The combined extracts were dried (MgSO₄) and concentrated, then the residue was dissolved in methanol and treated with 1M HCl in diethyl ether (0.6ml). The mixture was evaporated and the resulting solid recrystallised twice from methanol-diethyl ether to afford the title compound (57.5mg, 35%); δ_{H} (360MHz, DMSO-d₆) 4.65 (2H, br s, PhCH₂N), 6.96 (1H, d, J 16.4Hz, CH=*CH*Ar), 7.38 (1H, m, ArH), 7.43 (4H, m, ArH), 7.47-7.53 (2H, m, ArH), 7.59 (1H, m, ArH), 7.78 (1H, m, ArH), 8.09 (1H, d, J 16.4Hz, C*H*=CHAr), 9.10 (1H, br s, NH), 9.64 (1H, br s, NH), 10.36 (1H, br s, NH); m/z (ES⁺) 271/273 ([M+H]⁺).

The following Examples were prepared in an analogous process to the one described above:

### EXAMPLE 43

### (E)-N-Benzyl-3-chlorocinnamamidine hydrochloride

δ_{H} (360MHz, DMSO-d₆) 4.63 (2H, d, J 4.4Hz, PhCH₂NH), 6.97 (1H, d, J 16.4Hz, CH=C*H*Ar), 7.35-7.43 (5H, m, ArH), 7.55 (3H, m, ArH), 7.65 (1H, s, ArH), 7.89 (1H, d, J 16.4Hz, C*H*=CHAr), 9.08 (1H, br s, NH), 9.55 (1H, br s, NH), 10.27 (1H, br s, NH); m/z (ES⁺) 271/273 ([M+H]⁺).

### EXAMPLE 44

### (E)-N-Benzyl-4-chlorocinnamamidine hydrochloride

δ_{H} (400MHz, DMSO-d₆) 4.61 (2H, d, J 5.8Hz, PhCH₂NH), 6.86 (1H, d, J 16.4Hz, CH=C*H*Ar), 7.33-7.45 (5H, m, ArH), 7.56-7.63 (4H, m, ArH), 7.85 (1H, d, J 16.4Hz, C*H*=CHAr), 8.98 (1H, br s, NH), 9.44 (1H, br s, NH), 10.16 (1H, br s, NH); m/z (ES⁺) 271/273 ([M+H]⁺).

### EXAMPLE 45

### (E)-N-Benzyl-4-fluorocinnamamidine hydrochloride

δ_{H} (360MHz, DMSO-d₆) 4.61 (2H, d, J 5.9Hz, ArCH₂N), 6.80 (1H, d, J 16.4Hz, C*H*=CHAr), 7.40 (7H, m, ArH), 7.67 (2H, m, ArH), 7.86 (1H, d, J 16.5Hz, CH=C*H*Ar), 9.40 (1H, br s, NH), 9.97 (1H, br s, NH), 10.1 (1H, br s, NH); m/z (ES⁺) 255 ([M+H]⁺).

### EXAMPLE 46

### (E)-N-Benzyl-4-methoxycinnamamidine hydrochloride

δ_{H} (360MHz, DMSO-d₆) 3.83 (3H, s, ArOCH₃), 4.60 (2H, d, J 5.9Hz, ArCH₂N), 6.69 (1H, d, J 16.3Hz, C*H*=CHAr), 7.06 (2H, d, J 8.8Hz, ArH), 7.41 (5H, m, ArH), 7.56 (2H, d, J 8.8Hz, ArH), 7.82 (1H, d, J 16.4Hz, CH=C*H*Ar); m/z (ES⁺) 267 ([M+H]⁺).

### EXAMPLE 47

### (E)-N-Benzyl-3-methoxycinnamamidine hydrochloride

δ_{H} (360MHz, DMSO-d₆) 3.81 (3H, s, ArOCH₃), 4.61 (2H, s, ArCH₂N), 6.88 (1H, d, J 16.5Hz, C*H*=CHAr), 7.41 (5H, m, ArH), 7.07 (1H, m, ArH), 7.16 (2H, m, ArH), 7.40 (6H, m ArH), 7.83 (1H, d, J 16.4Hz, CH=C*H*Ar); m/z (ES⁺) 267 ([M+H]⁺).

### EXAMPLE 48

### (E)-N-Benzyl-2-methoxycinnamamidine hydrochloride

δ_{H} (360MHz, DMSO-d₆) 3.90 (3H, s, ArOCH₃), 4.60 (2H, d, J 5.9Hz, ArCH₂N), 6.95 (1H, d, J 16.6Hz, C*H*=CHAr), 7.41 (5H, m, ArH), 7.06 (1H, t, J 7.4Hz, ArH), 7.16 (1H, d, J 8.3Hz, ArH), 7.45 (6H, m ArH), 7.98 (1H, d, J 16.6Hz, CH=*CH*Ar), 9.9 (1H, br s, NH), 9.40 (1H, br s, NH), 10.10 (1H, br s, NH); m/z (ES⁺) 267 ([M+H]⁺).

### EXAMPLE 49

### (E)-N-Benzyl-3,4-difluorocinnamamidine hydrochloride

δ_{H} (360MHz, DMSO-d₆) 4.62 (2H, s, ArCH₂N), 6.87 (1H, d, J 16.4Hz, C*H*=CHAr), 7.35-7.48 (6H, m, ArH), 7.57 (1H, m, ArH), 7.71 (1H, m, ArH), 7.82 (1H, d, J 16.4Hz, CH=*CH*Ar), 9.03 (1H, br s NH), 9.49 (1H, br s, NH), 10.22 (1H, br s, NH); m/z (ES⁺) 272 ([M+H]⁺).

### EXAMPLE 50

### (E)-N-Benzyl-3-cyclohexaneacrylamidine hydrochloride

δ_{H} (360MHz, DMSO-d₆) 1.07-1.35 (5H, m, AlkH), 1.62-1.74 (5H, m, AlkH), 2.19-2.26 (1H, m, AlkH), 4.53 (2H, s, ArCH₂N), 1.91 (2H, m, AlkH), 6.06 (1H, dd, J 16.2Hz, 1.1Hz, C*H*=CH*c*-Hex), 7.04 (1H, dd, J 16.2Hz, 6.6Hz, CH=C*Hc*-Hex), 7.33-7.46 (5H, m, ArH), 9.00 (1H, br s, NH), 9.21 (1H, br s, NH), 10.00 (1H, br s, NH); m/z (ES⁺) 243 ([M+H]⁺).

## Claims

1. Use of a compound of formula I, or a pharmaceutically acceptable salt or hydrate thereof: 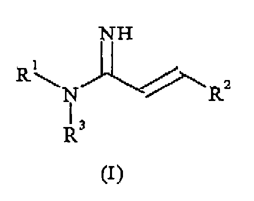 wherein:
R¹ represents C₁₋₆alkyl, C₃₋₇cycloalkyl, C₃₋₇cycloalkyl(C₁₋₆)alkyl, aryl, aryl(C₁₋₆)alkyl or heteroaryl(C₁₋₆)alkyl, any of which groups may be optionally substituted;
R² represents cyclopentyl, cyclohexyl, phenyl, naphthyl, pyridinyl, furyl, thienyl or pyrrolyl, any of which groups may be optionally substituted; and
R⁸ represents hydrogen or C₁₋₆ alkyl; or
R¹ and R³ are taken together with the intervening nitrogen atom to form an optionally substituted isoquinoline ring;
wherein the optional substituents on R¹, R² and R³ are one or two groups independently selected from C₁₋₆ alkyl, aryl, halogen, halo(C₁₋₆)alkyl, dihalo(C₁₋₆)alkyl, trihalo(C₁₋₆)alkyl, cyano, cyano(C₁₋₆)alkyl, hydroxy, hydroxymethyl, C₁₋₆ alkoxy, halo(C₁₋₆)alkoxy, dihalo(C₁₋₆)alkoxy and trihalo(C₁₋₆)alkoxy;
for the manufacture of a medicament for the treatment and/or prevention of neurological and/or neurodegenerative diseases.

2. A use as claimed in claim 1 wherein the compound is represented by formula IIA, and pharmaceutically acceptable salts or hydrates thereof: 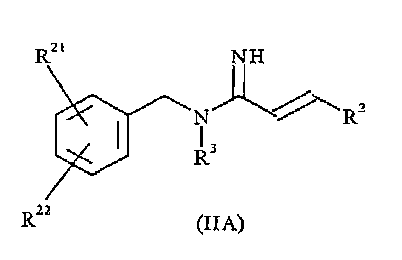 wherein R² and R³ are as defined in claim 1;
R²¹ represents hydrogen, C₁₋₆ alkyl, aryl, halogen, trihalo(C₁₋₆)alkyl, hydroxy, C₁₋₆alkoxy or trihalo(C₁₋₆)alkoxy; and
R²² represents hydrogen or halogen.

3. A use as claimed in claim 1 wherein the compound is represented by formula IIB, and pharmaceutically acceptable salts or hydrates thereof: 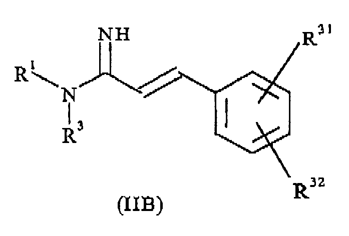 wherein R¹ and R³ are as defined in claim 1;
R³¹ represents hydrogen, C₁₋₆ alkyl, halogen or C₁₋₆ alkoxy; and
R³² represents hydrogen or halogen.

4. A use as claimed in any one of the preceding claims wherein the compound is represented by formula IIC, and pharmaceutically acceptable salts or hydrates thereof: 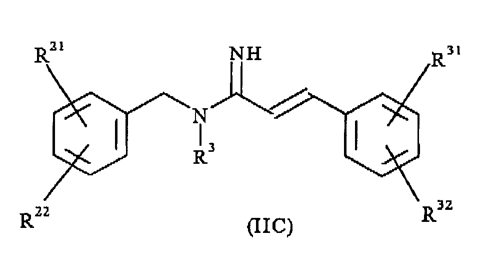 wherein R³ is as defined in claim 1;
R²¹ and R²² are as defined in claim 2; and
R³¹ and R³² are as defined in claim 3.

5. A use as claimed in claim 4 wherein the compound is represented by formula IID, and pharmaceutically acceptable salts or hydrates thereof: 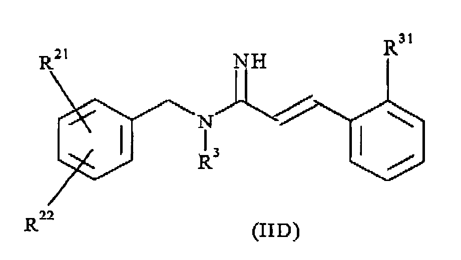 wherein R⁸ is as defined in claim 1;
R²¹ and R²² are as defined in claim 2; and
R³¹ is as defined in claim 3.

6. A use according to claim 1 wherein the compound is selected from:
(*E*)-N-(3-iodobenzyl)cinnamamidine;
(*E*)-N-(benzyl)cinnamamidine;
(*E*)-N-(4-chlorobenzyl)cinnamamidine;
(*E*)-N-(2-chlorobenzyl)cinnamamidine;
(*E*)-N-(3-chlorobenzyl)cinnamamidine;
(*E*)-N-(4-fluorobenzyl)cinnamamidine;
(*E*)-N-(4-trifluoromethylbenzyl)cinnamamidine;
(*E*)-N-(4-methoxybenzyl)cinnamamidine;
(*E*)-N-(4-methylbenzyl)cinnamamidine;
(*E*)-N-(3-methylbenzyl)cinnamamidine;
(*E*)-N-(2-methylbenzyl)cinnamamidine;
(*E*)-N-(3,4-dichlorobenzyl)cinnamamidine;
(*E*)-N-(2-phenylethyl)cinnamamidine;
(*E*)-N-(*R*)-α-methylbenzylcinnamamidine;
(*E*)-N-(*S*)-α-methylbenzylcinnamamidine;
(*E*)-N-(3-trifluoromethylbenzyl)cinnamamidine;
(*E*)-N-(3,5-dichlorobenzyl)cinnamamidine;
(*E*)-N-(2-methoxybenzyl)cinnamamidine;
(*E*)-N-(3-methoxybenzyl)cinnamamidine;
(*E*)-N-(2-trifluoromethylbenzyl)cinnamamidine;
(*E*)-N-(2,5-dichlorobenzyl)cinnamamidine;
(*E*)-N-(3-bromobenzyl)cinnamamidine;
(*E*)-N-(4-pyridylmethyl)cinnamamidine;
(*E*)-N-methyl-N-benzylcinnamamidine;
(*E*)-N-(2,3-dichlorobenzyl)cinnamamidine;
(*E*)-N-(cyclohexylmethyl)cinnamamidine;
(*E*)-N-(isobutyl)cinnamamidine;
(*E*)-N-(*n*-butyl)cinnamamidine;
(*E*)-N-(2-trifluoromethoxybenzyl)cinnamamidine;
(*E*)-N-(3-thienylmethyl)cinnamamidine;
(*E*)-N-(2-bromobenzyl)cinnamamidine;
(*E*)-N-(2-ethoxybenzyl)cinnamamidine;
(*E*)-N-(2-phenylbenzyl)cinnamamidine;
(*E*)-N-(2-furylmethyl)cinnamamidine;
(*E*)-N-(cyclohexyl)cinnamamidine;
(*E*)-N-(3-methyl-1-butyl)cinnamamidine;
(*E*)-2-(3-phenyl-1-imino-2-propenyl)-1,2,3,4-tetrahydiroisoquinoline;
(*E*)-N-(*n*-pentyl)cinnamamidine;
(*E*)-N-(3-phenyl-1-propyl)cinnamamidine;
(*E*)-N-(2-hydroxybenzyl)cinnamamidine;
(*E*)-N-phenylcinnamamidine;
(*E*)-N-benzyl-2-chlorocinnamamidine;
(*E*)-N-benzyl-3-chlorocinnamamidine;
(*E*)-N-benzyl-4-chlorocinnamamidine;
(*E*)-N-benzyl-4-fluorocinnamamidine;
(*E*)-N-benzyl-4-methoxycinnamamidine;
(*E*)-N-benzyl-3-methoxycinnamamidine;
(*E*)-N-benzyl-2-methoxycinnamamidine;
(*E*)-N-benzyl-3,4-difluorocinnamamidine;
(*E*)-N-benzyl-3-cyclohexaneacrylamidine;
and pharmaceutically acceptable salts and hydrates thereof.

7. A compound as defined in claim 2, 4, 5 or 6 or a pharmaceutically acceptable salt thereof.

8. A pharmaceutical composition comprising a compound of formula I as defined in claim 7 or a pharmaceutically acceptable salt or hydrate thereof in combination with a pharmaceutically acceptable carrier.

9. A compound as claimed in claim 7 or a pharmaceutically acceptable salt thereof for use in therapy.

10. A process for the preparation of a compound according to formula I as claimed in claim 1 which comprises:
(A) reacting a compound of formula III with a compound of formula IV: 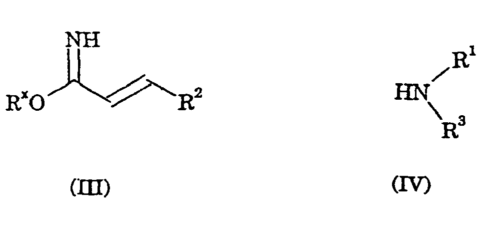 wherein R¹, R² and R³ are as defined in claim 1, and R^{x} represents C₁₋₆ alkyl; or
(B) reacting a compound of formula IV with a compound of formula VII: 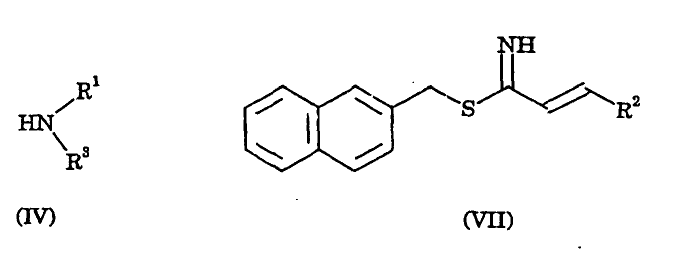 wherein R¹, R² and R³ are as defined in claim 1; or
(C) reacting a compound of formula XI with a compound of formula XII: 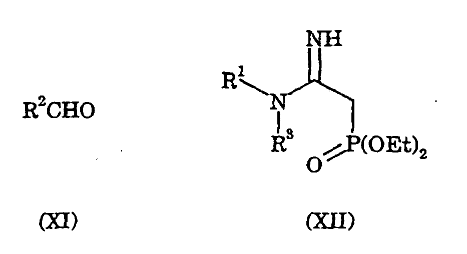 wherein R¹, R² and R³ are as defined in claim 1; and
(D) subsequently, if desired, converting a compound of formula I initially obtained into a further compound of formula I by standard methods.

## Patentansprüche

1. Verwendung einer Verbindung der Formel I oder eines pharmazeutisch verträglichen Salzes oder Hydrats davon: 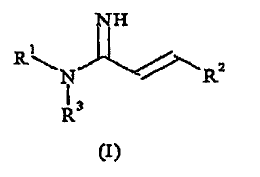 wobei:
R¹ C₁₋₆-Alkyl, C₃₋₇-Cycloalkyl, C₃₋₇-Cycloalkyl-(C₁₋₆)-alkyl, Aryl, Aryl-(C₁₋₆)-alkyl oder Heteroaryl-(C₁₋₆)-alkyl darstellt, jede von diesen Gruppen gegebenenfalls substituiert sein kann;
R² Cyclopentyl, Cyclohexyl, Phenyl, Naphthyl, Pyridinyl, Furyl, Thienyl oder Pyrrolyl darstellt, jede von diesen Gruppen gegebenenfalls substituiert sein kann; und
R⁸ Wasserstoff oder C₁₋₆-Alkyl darstellt; oder
R¹ und R³ mit dem dazwischentretenden Stickstoffatom zusammengenommen sind, wobei ein gegebenenfalls substituierter Isochinolinring erzeugt wird;
wobei die optionalen Substituenten an R¹, R² und R³ eine oder zwei Gruppen, unabhängig ausgewählt aus C₁₋₆-Alkyl, Aryl, Halogen, Halogen-(C₁₋₆)-alkyl, Dihalogen-(C₁₋₆)-alkyl, Trihalogen-(C₁₋₆)-alkyl, Cyano, Cyano-(C₁₋₆)-alkyl, Hydroxy, Hydroxymethyl, C₁₋₆-Alkoxy, Halogen-(C₁₋₆)-alkoxy, Dihalogen-(C₁₋₆)-alkoxy und Trihalogen-(C₁₋₆)-alkoxy, sind;
für die Herstellung eines Medikaments für die Behandlung und/oder Verhinderung von neurologischen und/oder neurodegenerativen Krankheiten.

2. Verwendung nach Anspruch 1, wobei die Verbindung durch Formel IIA und pharmazeutisch verträgliche Salze oder Hydrate davon dargestellt ist: 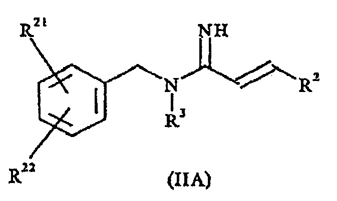 wobei R² und R³ wie in Anspruch 1 definiert sind;
R²¹ Wasserstoff, C₁₋₆-Alkyl, Aryl, Halogen, Trihalogen-(C₁₋₆)-alkyl, Hydroxy, C₁₋₆-Alkoxy oder Trihalogen-(C₁₋₆)-alkoxy darstellt; und
R²² Wasserstoff oder Halogen darstellt.

3. Verwendung nach Anspruch 1, wobei die Verbindung durch Formel IIB und pharmazeutisch verträgliche Salze oder Hydrate davon dargestellt ist: 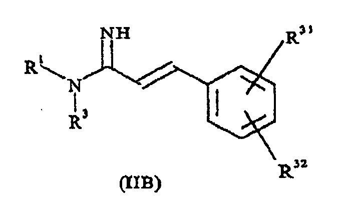 wobei R¹ und R³ wie in Anspruch 1 definiert sind;
R³¹ Wasserstoff, C₁₋₆-Alkyl, Halogen oder C₁₋₆-Alkoxy darstellt; und
R³² Wasserstoff oder Halogen darstellt.

4. Verwendung nach einem der vorhergehenden Ansprüche, wobei die Verbindung durch Formel IIC und pharmazeutisch verträgliche Salze oder Hydrate davon dargestellt ist: 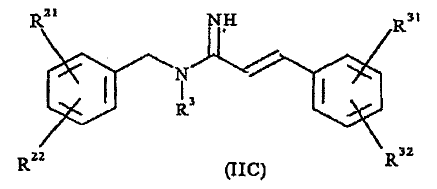 wobei R³ wie in Anspruch 1 definiert ist;
R²¹ und R²² wie in Anspruch 2 definiert sind; und
R³¹ und R³² wie in Anspruch 3 definiert sind.

5. Verwendung nach Anspruch 4, wobei die Verbindung durch Formel IID und pharmazeutisch verträgliche Salze oder Hydrate davon dargestellt ist: 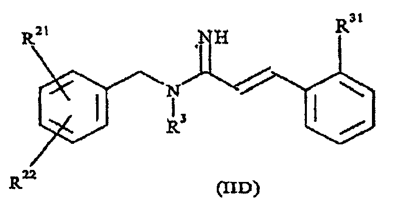 wobei R³ wie in Anspruch 1 definiert ist;
R²¹ und R²² wie in Anspruch 2 definiert sind; und
R³¹ wie in Anspruch 3 definiert ist.

6. Verwendung gemäß Anspruch 1, wobei die Verbindung aus:
(*E*)-N-(3-Iodbenzyl)cinnamamidin;
(*E*)-N-(Benzyl)cinnamamidin;
(*E*)-N-(4-Chlorbenzyl)cinnamamidin;
(*E*)-N-(2-Chlorbenzyl)cinnamamidin;
(*E*)-N-(3-Chlorbenzyl)cinnamamidin;
(*E*)-N-(4-Fluorbenzyl)cinnamamidin;
(*E*)-N-(4-Trifluormethylbenzyl)cinnamamidin;
(*E*)-N-(4-Methoxybenzyl)cinnamamidin;
(*E*)-N-(4-Methylbenzyl)cinnamamidin;
(*E*)-N-(3-Methylbenzyl)cinnamamidin;
(*E*)-N-(2-Methylbenzyl)cinnamamidin;
(*E*)-N-(3,4-Dichlorbenzyl)cinnamamidin;
(*E*)-N-(2-Phenylethyl)cinnamamidin;
(*E*)-N-(*R*)-α-Methylbenzylcinnamamidin;
(*E*)-N-(*S*)-α-Methylbenzylcinnamamidin;
(*E*)-N-(3-Trifluormethylbenzyl)cinnamamidin;
(*E*)-N-(3,5-Dichlorbenzyl)cinnamamidin;
(*E*)-N-(2-Methoxybenzyl)cinnamamidin;
(*E*)-N-(3-Methoxybenzyl)cinnamamidin;
(*E*)-N-(2-Trifluormethylbenzyl)cinnamamidin;
(*E*)-N-(2,5-Dichlorbenzyl)cinnamamidin;
(*E*)-N-(3-Brombenzyl)cinnamamidin;
(*E*)-N-(4-Pyridylmethyl)cinnamamidin;
(*E*)-N-Methyl-N-benzylcinnamamidin;
(*E*)-N-(2,3-Dichlorbenzyl)cinnamamidin;
(*E*)-N-(Cyclohexylmethyl)cinnamamidin;
(*E*)-N-(Isobutyl)cinnamamidin;
(*E*)-N-(n-Butyl)cinnamamidin;
(*E*)-N-(2-Trifluormethoxybenzyl)cinnamamidin;
(*E*)-N-(3-Thienylmethyl)cinnamamidin;
(*E*)-N-(2-Brombenzyl)cinnamamidin;
(*E*)-N-(2-Ethoxybenzyl)cinnamamidin;
(*E*)-N-(2-Phenylbenzyl)cinnamamidin;
(*E*)-N-(2-Furylmethyl)cinnamamidin;
(*E*)-N-(Cyclohexyl)cinnamamidin;
(*E*)-N-(3-Methyl-1-butyl)cinnamamidin;
(*E*)-N-2-(3-Phenyl-1-imino-2-propenyl)-1,2,3,4-tetrahydroisochinolin;
(*E*)-N-(*n*-Pentyl)cinnamamidin;
(*E*)-N-(3-Phenyl-1-propyl)cinnamamidin;
(*E*)-N-(2-Hydroxybenzyl)cinnamamidin;
(*E*)-N-Phenylcinnamamidin;
(*E*)-N-Benzyl-2-chlorcinnamamidin;
(*E*)-N-Benzyl-3-chlorcinnamamidin;
(*E*)-N-Benzyl-4-chlorcinnamamidin;
(*E*)-N-Benzyl-4-fluorcinnamamidin;
(*E*)-N-Benzyl-4-methoxycinnamamidin;
(*E*)-N-Benzyl-3-methoxycinnamamidin;
(*E*)-N-Benzyl-2-methoxycinnamamidin;
(*E*)-N-Benzyl-3,4-difluorcinnamamidin;
(*E*)-N-Benzyl-3-cyclohexanacrylamidin;
und pharmazeutisch verträglichen Salzen oder Hydraten davon ausgewählt ist.

7. Verbindung wie in Anspruch 2, 4, 5 oder 6 definiert oder ein pharmazeutisch verträgliches Salz davon.

8. Pharmazeutische Zusammensetzung, umfassend eine Verbindung der Formel I, wie in Anspruch 7 definiert, oder ein pharmazeutisch verträgliches Salz oder Hydrat davon in Kombination mit einem pharmazeutisch verträglichen Träger.

9. Verbindung nach Anspruch 7 oder ein pharmazeutisch verträgliches Salz davon zur Verwendung in der Therapie.

10. Verfahren für die Herstellung einer Verbindung gemäß Formel I nach Anspruch 1, welches umfaßt:
(A) Umsetzen einer Verbindung der Formel III mit einer Verbindung der Formel IV: 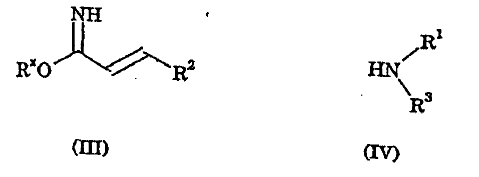 wobei R¹, R² und R³ wie in Anspruch 1 definiert sind und R^{x} C₁₋₆-Alkyl darstellt; oder
(B) Umsetzen einer Verbindung der Formel IV mit einer Verbindung der Formel VII: 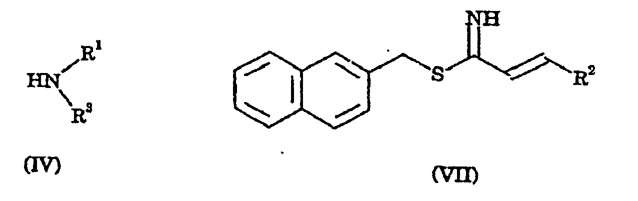 wobei R¹, R² und R³ wie in Anspruch 1 definiert sind; oder
(C) Umsetzen einer Verbindung der Formel XI mit einer Verbindung der Formel XII: 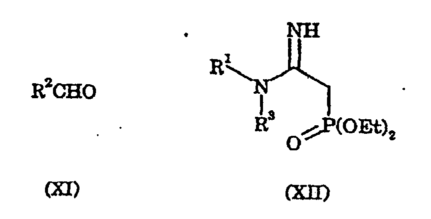 wobei R¹, R² und R³ wie in Anspruch 1 definiert sind; und
(D) nachfolgend, wenn gewünscht, Umwandeln einer anfänglich erhaltenen Verbindung der Formel I in eine weitere Verbindung der Formel I vermittels Standardverfahren.

## Revendications

1. Utilisation d'un composé de formule I, ou d'un sel ou hydrate pharmaceutiquement acceptable de celui-ci: 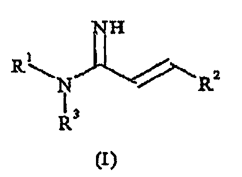 dans lequel:
R¹ représente alkyle en C₁₋₆, cycloalkyle en C₃₋₇, cycloalkyl en C₃₋₇-alkyle en (C₁₋₆), aryle, arylalkyle en (C₁₋₆) ou hétéroaryl-alkyle en (C₁₋₆), tous groupes parmi ceux-ci pouvant être facultativement substitués;
R² représente cyclopentyle, cyclohexyle, phényle, naphtyle, pyridinyle, furyle, thiényle ou pyrrolyle, tous groupes parmi ceux-ci pouvant être facultativement substitués; et
R⁸ représente de l'hydrogène ou alkyle en C₁₋₆; ou
R¹ et R² sont pris ensemble avec l'atome d'azote intermédiaire pour former un noyau isoquinoléine facultativement substitué;
dans lequel les substituants facultatifs sur R¹, R² et R³ sont un ou deux groupes indépendamment choisis parmi alkyle en C₁₋₆, aryle, halogène, halogéno-alkyle en (C₁₋₆), dihalogéno-alkyle en (C₁₋₆), trihalogéno-alkyle en (C₁₋₆), cyano, cyano-alkyle en (C₁₋₆), hydroxy, hydroxyméthyle, alcoxy en C₁₋₆, halogéno-alcoxy en (C₁₋₆), dihalogéno-alcoxy en (C₁₋₆) et trihalogéno-alcoxy en (C₁₋₆);
pour la fabrication d'un médicament pour le traitement et/ou la prévention de maladies neurologiques et/ou neurodégénératives.

2. Utilisation selon la revendication 1, dans laquelle le composé est représenté par la formule IIA, et les sels ou hydrates pharmaceutiquement acceptables de celui-ci: 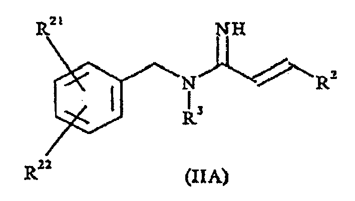 dans laquelle R² et R³ sont tels que définis dans la revendication 1;
R²¹ représente de l'hydrogène, alkyle en C₁₋₆, aryle, halogène, trihalogéno-alkyle en (C₁₋₆), hydroxy, alcoxy en C₁₋₆ ou trihalogéno-alcoxy en (C₁₋₆); et
R²² représente de l'hydrogène ou un halogène.

3. Utilisation selon la revendication 1, dans laquelle le composé est représenté par la formule IIB, et les sels ou hydrates pharmaceutiquement acceptables de celui-ci: 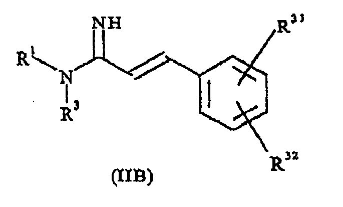 dans laquelle R¹ et R³ sont tels que définis dans la revendication 1;
R³¹ représente de l'hydrogène, alkyle en C₁₋₆, halogène ou alcoxy en C₁₋₆; et
R³² représente de l'hydrogène ou un halogène.

4. Utilisation selon l'une quelconque des revendications précédentes, dans laquelle le composé est représenté par la formule IIC, et les sels ou hydrates pharmaceutiquement acceptables de celui-ci: 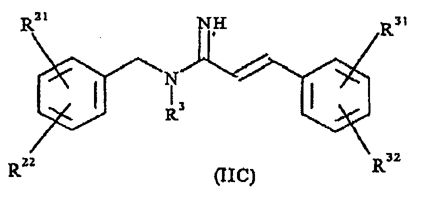 dans laquelle R³ est tel que défini dans la revendication 1;
R²¹ et R²² sont tels que définis dans la revendication 2; et
R³¹ et R³² sont tels que définis dans la revendication 3.

5. Utilisation selon la revendication 4, dans laquelle le composé est représenté par la formule IID, et les sels ou hydrates pharmaceutiquement acceptables de celui-ci: 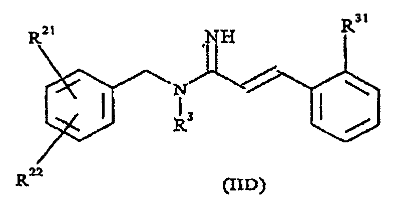 dans laquelle R³ est tel que défini dans la revendication 1;
R²¹ et R²² sont tels que définis dans la revendication 2; et
R³¹ est tel que défini dans la revendication 3.

6. Utilisation selon la revendication 1, dans laquelle le composé est choisi parmi:
la (*E*)-N-(3-iodobenzyl)cinnamamidine;
la (*E*)-N-(benzyl)cinnamamidine;
la (*E*)-N-(4-chlorobenzyl)cinnamamidine;
la (*E*)-N-(2-chlorobenzyl)cinnamamidine;
la (*E*)-N-(3-chlorobenzyl)cinnamamidine;
la (*E*)-N-(4-fluorobenzyl)cinnamamidine;
la (*E*)-N-(4-trifluorométhylbenzyl)cinnamamidine;
la (*E*)-N-(4-méthoxybenzyl)cinnamamidine;
la (*E*)-N-(4-méthylbenzyl)cinnamamidine;
la (*E*)-N-(3-méthylbenzyl)cinnamamidine;
la (*E*)-N-(2-méthylbenzyl)cinnamamidine;
la (*E*)-N-(3,4-dichlorobenzyl)cinnamamidine;
la (*E*)-N-(2-phényléthyl)cinnamamidine;
la (*E*)-N-(*R*)-α-méthylbenzylcinnamamidine;
la (*E*)-N-(*S*)-α-méthylbenzylcinnamamidine;
la (*E*)-N-(3-trifluorométhylbenzyl)cinnamamidine;
la (*E*)-N-(3,5-dichlorobenzyl)cinnamamidine;
la (*E*)-N-(2-méthoxybenzyl)cinnamamidine;
la (*E*)-N-(3-méthoxybenzyl)cinnamamidine;
la (*E*)-N-(2-trifluorométhylbenzyl)cinnamamidine;
la (*E*)-N-(2,5-dichlorobenzyl)cinnamamidine;
la (*E*)-N-(3-bromobenzyl)cinnamamidine;
la (*E*)-N-(4-pyridylméthyl)cinnamamidine;
la (*E*)-N-méthyl-N-benzylcinnamamidine;
la (*E*)-N-(2,3-dichlorobenzyl)cinnamamidine;
la (*E*)-N-(cyclohexylméthyl)cinnamamidine;
la (*E*)-N-(isobutyl)cinnamamidine;
la (*E*)-N-(*n*-butyl)cinnamamidine;
la (*E*)-N-(2-trifluorométhoxybenzyl)cinnamamidine;
la (*E*)-N-(3-thiénylméthyl)cinnamamidine;
la (*E*)-N-(2-bromobenzyl)cinnamamidine;
la (*E*)-N-(2-éthoxybenzyl)cinnamamidine;
la (*E*)-N-(2-phénylbenzyl)cinnamamidine;
la (*E*)-N-(2-furylméthyl)cinnamamidine;
la (*E*)-N-(cyclohexyl)cinnamamidine;
la (*E*)-N-(3-méthyl-1-butyl)cinnamamidine;
la (*E*)-2-(3-phényl-1-imino-2-propényl)-1,2,3,4-tétrahydroisoquinoléine;
la (*E*)-N-(*n*-pentyl)cinnamamidine;
la (*E*)-N-(3-phényl-1-propyl)cinnamamidine;
la (*E*)-N-(2-hydroxybenzyl)cinnamamidine;
la (*E*)-N-phénylcinnamamidine;
la (*E*)-N-benzyl-2-chlorocinnamamidine;
la (*E*)-N-benzyl-3-chlorocinnamamidine;
la (*E*)-N-benzyl-4-chlorocinnamamidine;
la (*E*)-N-benzyl-4-fluorocinnamamidine;
la (*E*)-N-benzyl-4-méthoxycinnamamidine;
la (*E*)-N-benzyl-3-méthoxycinnamamidine;
la (*E*)-N-benzyl-2-méthoxycinnamamidine;
la (*E*)-N-benzyl-3,4-difluorocinnamamidine;
la (*E*)-N-benzyl-3-cyclohexaneacrylamidine;
et les sels et hydrates pharmaceutiquement acceptables de celui-ci.

7. Composé tel que défini dans la revendication 2, 4, 5 ou 6 ou sel pharmaceutiquement acceptable de celui-ci.

8. Composition pharmaceutique comprenant un composé de formule 1 tel que défini dans la revendication 7 ou un sel ou hydrate pharmaceutiquement acceptable de celui-ci en combinaison avec un véhicule pharmaceutiquement acceptable.

9. Composé selon la revendication 7 ou sel pharmaceutiquement acceptable de celui-ci pour l'utilisation en thérapie.

10. Procédé de préparation d'un composé selon la formule 1 tel que revendiqué dans la revendication 1, qui comprend:
(A) la réaction d'un composé de formule III avec un composé de formule IV: 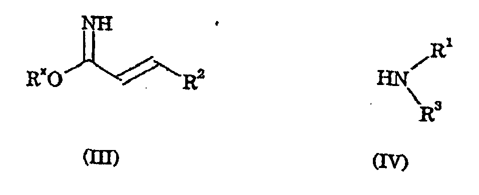 où R¹, R² et R³ sont tels que définis dans la revendication 1, et R^{x} représente alkyle en C₁₋₆; ou
(B) la réaction d'un composé de formule IV avec un composé de formule VII: 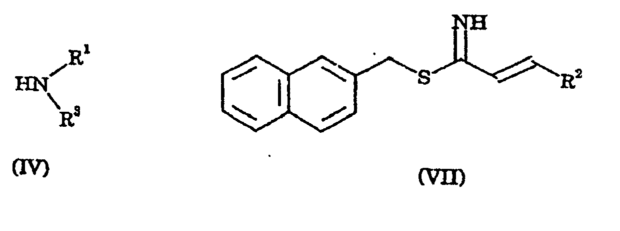 où R¹, R² et R³ sont tels que définis dans la revendication 1; ou
(C) la réaction d'un composé de formule XI avec un composé de formule XII: 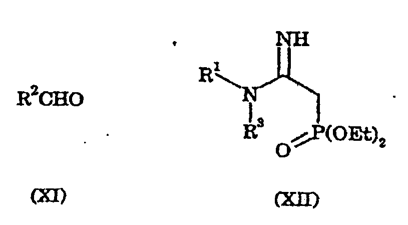 où R¹, R² et R³ sont tels que définis dans la revendication 1; et
(D) ensuite, si on le souhaite, la conversion d'un composé de formule I initialement obtenu en un autre composé de formule I par des procédés standards.
